# EUROPEAN PATENT SPECIFICATION

(11) **EP 3 846 801 B1**
(45) Date of publication and mention of the grant of the patent: **12.03.2025**
(21) Application number: 19762831.6
(22) Date of filing: 09.09.2019
(51) Int. Cl.: A61K 31/404, A61K 31/427, A61K 31/443, A61P 9/10, A61P 25/28, A61P 43/00

(54) **USE OF CFTR MODULATORS FOR TREATING CEREBROVASCULAR CONDITIONS**
VERWENDUNG VON CFTR-MODULATOREN ZUR BEHANDLUNG VON ZEREBROVASKULÄREN ZUSTÄNDEN
UTILISATION DE MODULATEURS DU CFTR POUR TRAITER DES AFFECTIONS CÉRÉBROVASCULAIRES

(30) Priority: 09.09.2018 EP 18193319
(43) Date of publication of application: 14.07.2021
(73) Proprietor: Qanatpharma AG, 6370 Stans (CH)
(72) Inventor: BOLZ, Steffen-Sebastian, 82211 Herrsching (DE)
(74) Representative: Habermann, Hruschka & Schnabel
(86) International application number: PCT/EP2019/074011
(87) International publication number: WO 2020/049189

(56) References cited:
- WO-A1-2016/105484
- WO-A2-2010/068863
- US-A1- 2005 245 433
- DARCY LIDINGTON ET AL: "Cerebral artery myogenic reactivity: The next frontier in developing effective interventions for subarachnoid hemorrhage", JOURNAL OF CEREBRAL BLOOD FLOW & METABOLISM, vol. 38, no. 1, 14 November 2017 (2017-11-14), US, pages 17 - 37, XP055645544, ISSN: 0271-678X, DOI: 10.1177/0271678X17742548

## Description

The present invention relates to the use of the CFTR modulators lumacaftor or tezacaftor for use in preventing and/or treating cerebrovascular conditions.

In an aging world population, the number of individuals living with heart disease is rapidly growing. The underlying causes are higher incidence rates in the elderly and medical advancements that have significantly improved survival rates. Over the last decade, it became apparent that the extension in longevity is associated with a significant cognitive decline (1) and feeds an emerging "silent epidemic" of cognitive impairment (2). The impact will be substantial: in addition to direct medical needs, cognitive impairment seriously impacts the patients' ability to cope with their primary disease (e.g., reduced treatment compliance) and has significant social effects on families, friends and society at large. The true burden of this emerging epidemic, therefore, cannot be quantified in simple economic figures. What is certain is that our medical and mental health networks are not structurally or financially equipped to accommodate the impending crisis.

Cognitive decline in heart failure (HF) patients is usually attributed to "vascular cognitive impairment", a term that describes multiple forms of cognitive impairment (e.g., memory loss, loss of executive function, confusion) that arise from a vascular origin. As an umbrella term, the etiology of vascular cognitive impairment is diverse and multi-factorial; the clinical diagnosis is challenging and imprecise; and there are multiple distinct and complex injury sub-types (1). Most cardiovascular and cerebrovascular pathologies (including hypertension, ischemic and hemorrhagic stroke, heart disease and diabetes) are considered primary and significant causes of vascular-based cognitive decline (1). Although vascular cognitive impairment encompasses many complex casual mechanisms, the unifying aspect is insufficient cerebral perfusion: in this regard, recent work by the inventors has focused on improving cerebral perfusion in experimental models of HF and subarachnoid hemorrhage (SAH) (3-6).

The problem underlying the present invention is to provide a novel regimen for improving cerebrovascular conditions.

The solution to the above technical problem is provided by the embodiments of the present invention as disclosed in the claims, the present description and the accompanying figures.

The scope of the invention is defined by the claims. Any information disclosed herein but not covered by the claims is included for information only.

All references to methods of treatment of the human or animal body in the present description are to be interpreted as references to the compounds of the present invention for use in a method for treatment of the human (or animal) body by therapy.

The inventors uncovered key molecular mechanisms that reduce cerebral perfusion in HF and SAH (4-6). Remarkably, both pathologies alter cerebrovascular autoregulation and hence, cerebral blood flow (CBF), by the same fundamental mechanism. Specifically, HF and SAH induce robust tumor necrosis factor (TNF) expression within the cerebral artery vascular wall: smooth muscle cell-localized TNF acts by an autocrine/paracrine mechanism to stimulate sphingosine-1-phosphate (S1P) production, which then elicits vasoconstriction via the S1P₂ receptor subtype (3,4,6). The inventors capitalized on this mechanistic insight and successfully validated two therapeutic interventions: sequestering TNF (etanercept) or antagonizing S1P₂ receptor signaling in both models eliminates the HF- and SAH-mediated enhancement of cerebrovascular vasoconstriction (i.e., measured as augmented myogenic tone) (3,4,6), normalizes cerebral perfusion (3,4,6) and consequently, reduces neuronal injury (5,6). However, the utility of these two interventions may be limited: etanercept carries significant risks stemming from immunosuppression (7) and may compromise blood pressure control through effects on skeletal muscle resistance arteries (8); while antagonizing S1P₂ receptors will undoubtedly elicit unpredictable secondary effects, as these receptors mediate important and diverse functions in several organs (9).

In searching alternative therapeutic targets, the cystic fibrosis transmembrane conductance regulator (CFTR) emerged as an intriguing candidate. Previous work by the inventors demonstrated that HF and SAH down-regulate CFTR protein expression in cerebral arteries: since CFTR is a critical transporter involved in smooth muscle cell S1P degradation (4), CFTR down-regulation should enhance S1P bioavailability and its pro-constrictive effects (3,4). Indeed, vascular TNF expression, CFTR expression and myogenic reactivity are tightly correlated (3,4,6), fueling the mechanistic proposal according to the invention that the TNF-dependent augmentation of cerebral artery myogenic tone is mediated through reduced CFTR protein expression (4). In this context, CFTR becomes an attractive and logical therapeutic target for correcting myogenic reactivity within the cerebral microcirculation, since CFTR therapeutics are not known to compromise immune function and FDA-approved therapeutics that increase CFTR expression/activity are available (10).

WO 2016/105484 and WO 2010/068863 disclose CFTR modulators, and mention that they are useful in the treatment of various conditions including Alzheimer's diseases and diabetes.

DARCY LIDINGTON ET AL: "Cerebral artery myogenic reactivity: The next frontier in developing effective interventions for subarachnoid hemorrhage",JOURNAL OF CEREBRAL BLOOD FLOW & METABOLISM, vol. 38, no. 1, 14 November 2017 (2017-11-14), pages 17-37 speculates that CFTR modulators may be useful in the treatment of subarachnoid hemorrhage.

The present invention provides the use of CFTR modulator compounds. i.e. according to the invention there may be used one or more CFTR modulators, selected from lumacaftor, tezacaftor or pharmaceutically acceptable salts and solvates thereof, for treating and/or preventing a cerebrovascular condition.

According to the invention a "cerebrovascular condition" is a condition that reduces, impairs or otherwise afflicts the cerebrovascular system of an individual, preferably a human patient. More particular, the cerebrovascular condition is reduced or impaired cerebrovascular perfusion. The term "cerebrovascular perfusion" is also known as, and synonymous to, respectively, "cerebral blood flow" and "cerebral perfusion", respectively.

According to the invention, the CFTR modulator exerts its effect on the CFTR proteins present or expressed, respectively, in cerebrovascular smooth muscle cells. In doing so the CFTR modulator normalize cerebrovascular Erk1/2 phosphorylation and sphingosine-1-phosphate signalling and/or cerebrovascular myogenic reactivity and/or cerebrovascular blood flow and/or neuronal apoptosis and cerebrovascular damage and/or correct behavioural and learning deficits in patients with cerebrovascular conditions, preferably reduced or impaired, respectively, cerebrovascular perfusion.

Typically, the patient having a cerebrovascular condition or disease state, respectively, has a primary diseases state, typically an underlying disease which in turn causes said cerebrovascular condition, or at least a disease state which associated with said cerebrovascular condition. According to the invention, preferred cerebrovascular conditions are treated or prevented, respectively, with said one or more CFTR modulators, which condition(s) is/are caused by heart disease, heart failure (HF), subarachnoid haemorrhage (SAH), sudden sensoneurinal hearing loss (SSHL), vascular dementia, hypertension, ischemic stroke, hemorrhagic stroke, heart disease and diabetes and Alzheimer's disease. A "CFTR modulator" according to the invention is a compound modulating the function of the CFTR protein so that it can serve its primary function, namely, to create a channel for chloride (a component of salt) to flow across the cell surface.

Several CFTR modulators are known in the art, and preferably include CFTR potentiators, CFTR correctors and CFTR amplifiers. CFTR potentiators increase CFTR channel activity. CFTR correctors increase CFTR cell surface expression. CFTR amplifiers increase the amount of expressed CFTR by increasing the amount of CFTR mRNA. More preferred CFTR modulators of the invention are drugs having a proteostatic effect on CFTR, in particular CFTR correctors and CFTR amplifiers, particularly preferred CFTR correctors. CFTR correctors are also highly preferred for use in the invention because they exert a very good activity even when CFTR abundance is low.

CFTR corrector compounds disclosed herein are cyclopropane carboxamide derivatives such as those disclosed in WO-A-2005/075435, WO-A-2007/021982, WO-A-2008/127399, WO-A-2009/108657 and WO-A-2009/123896. Other CFTR corrector compounds are aminoheterocyclyl derivatives, such as pyrimidine derivatives, preferably compounds disclosed in WO-A-2010/068863, WO-A-2010/151747 and WO-A-2011/008931, aminothiazole derivatives, preferably compounds disclosed in WO-A-2006/101740, and quinoline/quinazoline derivatives, preferably compounds disclosed in WO-A-2012/166654, coumarin derivatives, preferably compounds disclosed in WO-A-2014/152213, and trimethyl angelicin derivatives, preferably compounds disclosed in WO-A-2012/171954.Further useful CFTR corrector compounds are disclosed in WO-A-2014/081821, WO-A-2014/081820 and WO-A-2010/066912. Further CFTR corrector compounds and compositions thereof include tranglutaminase 2 (TG2) inhibitors and casein kinase 2 (CK2) inhibitors. A preferred TG2 inhibitor is cysteamine. A preferred CK2 inhibitor is epigallocatechin gallate (ECGC). According to an embodiment also disclosed but not part of the invention, a TG2 inhibitor, preferably, cysteamine, and a CK2 inhibitor, preferably ECGC, are used in combination. Such combination may be embodied as a simultaneous administration, preferably in a single composition, but simultaneous administration of the two individual inhibitors, namely a TG2 inhibitor and a CK2 inhibitor, each inhibitor either as such or as a component of a pharmaceutical composition, is also envisaged. In an alternative embodiment, the TG2 inhibitor and the CK2 inhibitor can also be administered sequentially (again, either as such or contained in a suitable composition. With respect to the combination of a TG2 inhibitor and a CK2 inhibitor, preferably cysteamine and ECGC it is also referred to US-A-2013/0310329.

Specific examples of CFTR modulators include, C18 (VRT-534), lumacaftor (VX-809), tezacaftor (VX-661), 4,4',6-trimethyl angelicin, VRT-768, VRT-422, VRT-325, CFpot-532, Copo-22, 002_NB_28 (DBM228), DBM_003_8CI (DBM308) as well as any combination of such compounds. CFTR modulators according to the invention are tezacaftor and lumacaftor. It is to be understood that the present invention is also directed to the use of pharmaceutically acceptable salts, solvates, esters salts of such esters, as well as any other adduct or derivative which upon administration to a patient in need is capable of providing, directly or indirectly, a CFTR modulator for use in the invention or a metabolite or residue thereof.

The present invention is also directed to the CFTR modulator(s) lumacaftor or tezacaftor as described above for use in the treatment and/or prevention of the above-described conditions.

Furthermore, the present invention provides a method for the prevention and/or treatment of the above-described conditions by administering at least one (i.e. one or more) CFTR modulator to a patient in need of such treatment, preferably a human patient, more preferably a patient suffering from a cerebrovascular condition, associated with or caused by, respectively, one or more conditions selected from heart disease, heart failure (HF), subarachnoid haemorrhage (SAH), sudden sensoneurinal hearing loss (SSHL), vascular dementia, hypertension, ischemic stroke, hemorrhagic stroke, heart disease and diabetes and Alzheimer's disease.

According to the invention the one or more CFTR modulator(s) can be used in its/their free form. In other embodiments the CFTR modulator(s) (i.e. at least one CFTR modulator) as used in the present invention is present in a pharmaceutical composition comprising said at least one CFTR modulator typically in combination with at least one pharmaceutically acceptable excipient, diluent, carrier and/or vehicle.

The effective amount of the CFTR modulator to be applied in the method and uses of the invention, i.e. the specific effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts.

Preferred doses in the context of the invention, in particular with reference to lumacaftor and C18, are about 0.1 to about 10 mg per kg body weight (hereinafter referred to as "mg/kg"), preferably about 1 to about 8 mg/kg, more preferably about 3 to about 5 mg/kg. The dose may be administered once or more often, such as twice or thrice daily. Preferably, doses are administered once or twice daily.

The administration route for the CFTR modulator is not particularly critical and the chosen route depends on the individual CFTR modulator compound or compounds applied and the subject to be treated. Preferably the CFTR modulator(s) is/are administered systemically such as orally or by i.v. administration, which oral administration particularly preferred.

Topical application may also be considered with injection into the cerebrospinal fluid being a particularly preferred topical route.

The term "patient", as used herein, means an animal, preferably a mammal, and most preferably a human.

The at least one CFTR modulator, preferably present in a pharmaceutical composition as outlined above, for use according to the invention may be administered using any amount and any route of administration effective for treating the cerebrovascular condition. It is understood according to the invention that the term "treating" or "treatment" means that the severity of the condition does at least not progress as compared tot he non-treated condition, preferably the severity of the condition does not progress, more preferably, the severity of the condition is lessened, even more preferred substantially lessened, and, ideally, the condition is cured to a substantial extent. Preferably, the severity of the condition according to the invention is reduced at least by 30 %, more preferably by at least 50 %, particularly by at least 70 %, even more preferred by at least 90 %, with complete cure of the condition being the most preferred outcome of the inventive treatment.

With respect to the cerebrovascular condition being the target of the present use and method, respectively, the degree of lessening the severity or degree of curing, respectively, the cerebrovascular condition is typically assessed by neurological assessment of the treated subject according to the medical and diagnostic procedures known to the person skilled in the art, preferably before treatment according to the invention, preferably in typical intervals after onset of the inventive treatment. Such assessments include, preferably, MRI-based measurement of cerebral perfusion, e.g. as described in the below example (optionally adapted to the patient under examination, e.g. when the patient is a human patient).

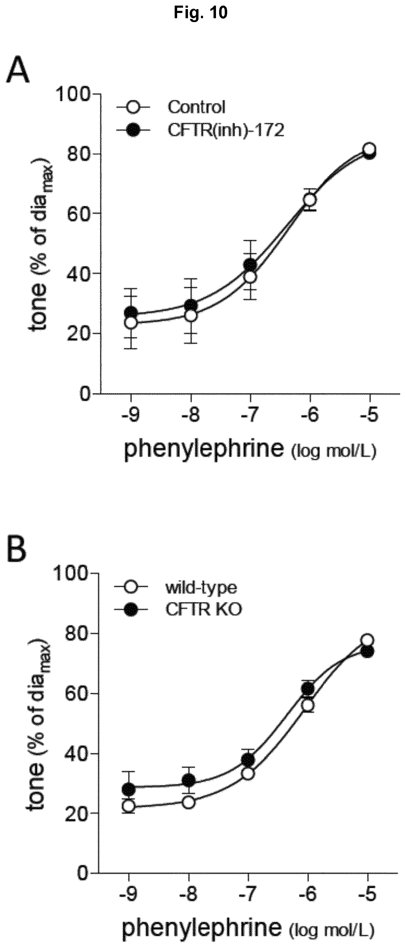

The figures show:

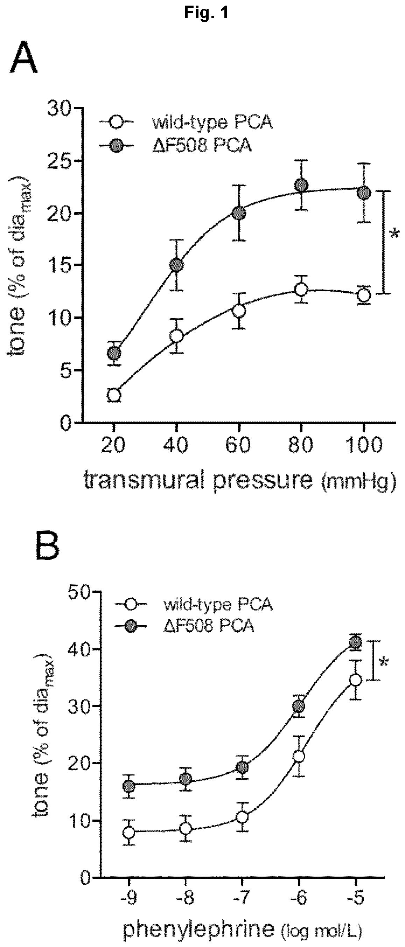

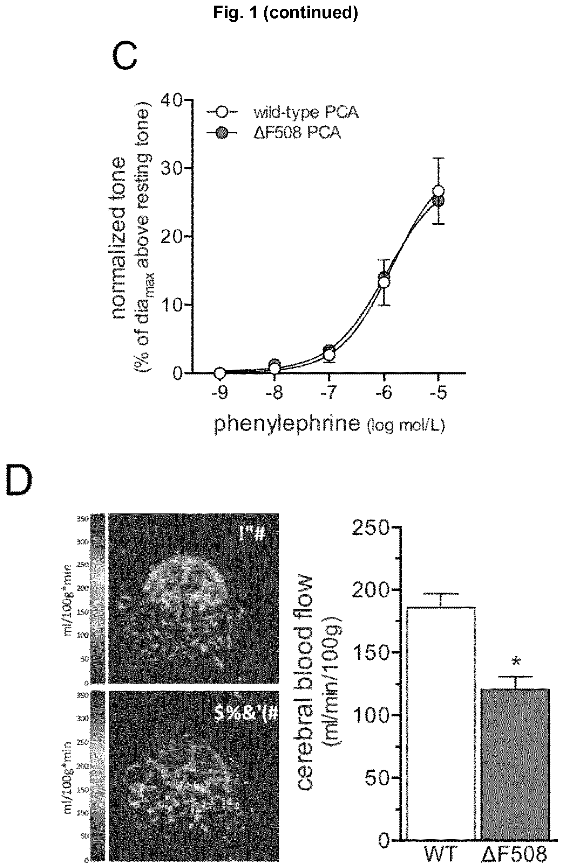

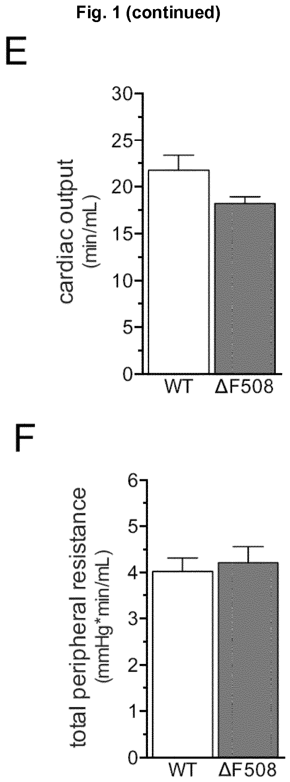

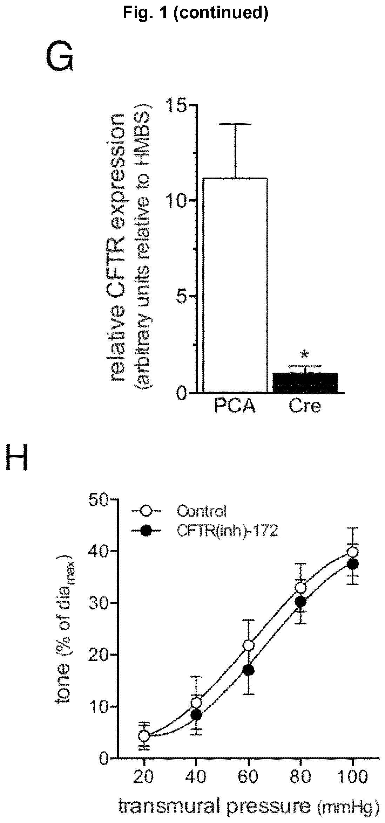

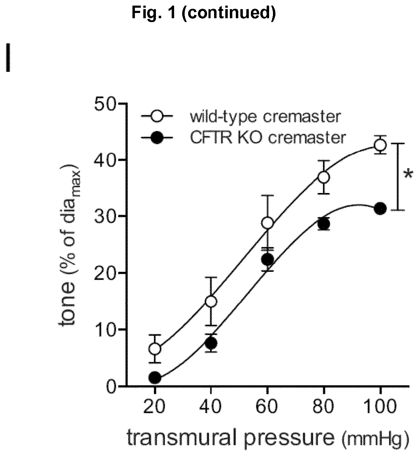

**Figure 1****. Cerebral blood flow is reduced in CFTR^{ΔF508} mice**
   **(A)** Myogenic vasoconstriction is stronger in posterior cerebral arteries (PCA) isolated from cystic fibrosis transmembrane conductance regulator (CFTR) ΔF508 mutant mice, relative to wild-type (WT) littermate controls. **(B)** Posterior cerebral arteries isolated from CFTR^{ΔF508} mice display an upward shift in their phenylephrine dose-response relationship. **(C)** However, once the phenylephrine responses are normalized to basal tone (tone_{active} - toneᵣₑₛₜ, where tone_{active} is the tone at given phenylephrine concentration and toneᵣₑₛₜ is the tone immediately prior to stimulation), the WT and CFTR^{ΔF508} phenylephrine dose-response relationships are virtually identical (Two-way ANOVA test P=N.S.). Mean maximal vessel diameters at 45 mmHg (diaₘₐₓ) are: CFTR^{ΔF508}: 186±2 µm, n=6 from 4 mice and WT: 169±8 µm, n=5 from 3 mice; (Mann-Whitney test P=N.S. for diaₘₐₓ). **(D)** Magnetic Resonance Imaging was used to measure cerebral blood flow in predefined forebrain cortical regions. Shown are representative perfusion maps from WT and CFTR^{ΔF508} mouse forebrains. Cerebral blood flow is significantly lower in CFTR^{.ΔF508} mice (n=10), relative to WT littermates (n=11); however, neither **(E)** cardiac output (n=5 for both groups) nor **(F)** total peripheral resistance (n=5 for both groups) differed between the two genotypes. **(G)** In WT mice, CFTR mRNA expression is significantly higher in posterior cerebral arteries (n=5), relative to cremaster skeletal muscle arteries (Cre; n=6). **(H)** Cremaster skeletal muscle resistance artery myogenic tone is not altered by CFTR inhibition *in vitro* (100 nmol/L CFTR₍ᵢₙₕ₎-172 for 30 minutes). **(I)** CFTR gene deletion (CFTR KO), however, induces a modest, but significant attenuation of myogenic tone. Mean maximal vessel diameters at 60 mmHg (diaₘₐₓ) are: WT (*Panel G*)*:* 72±3 µm, n=5 from 4 mice; CFTR KO (*Panel H*)*:* 88±4 µm, n=6 from 4 mice; and WT littermates (*Panel H*)*:* 78±4 µm, n=5 from 2 mice. All data are mean ± s.e.m. * denotes P<0.05 for an unpaired comparison with a Mann-Whitney test. Abbreviations: CFTR - cystic fibrosis transmembrane conductance regulator; Cre - cremaster; KO - knockout; PCA - posterior cerebral artery; WT - wild type.

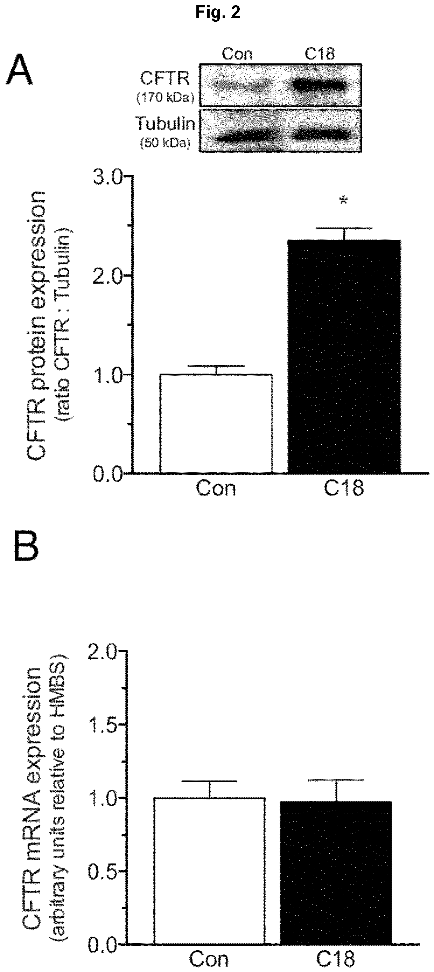

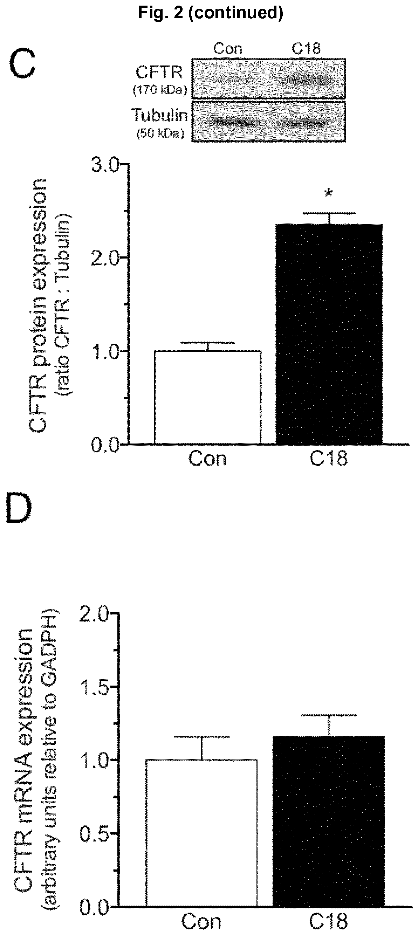

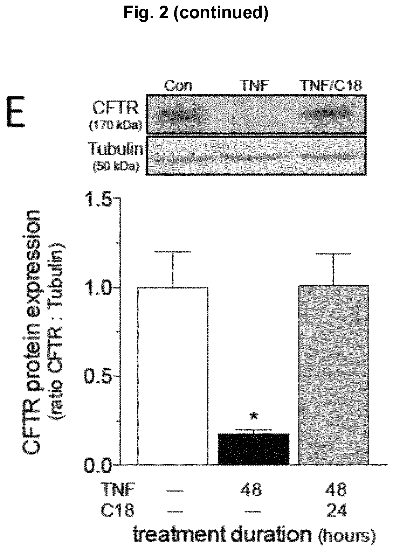

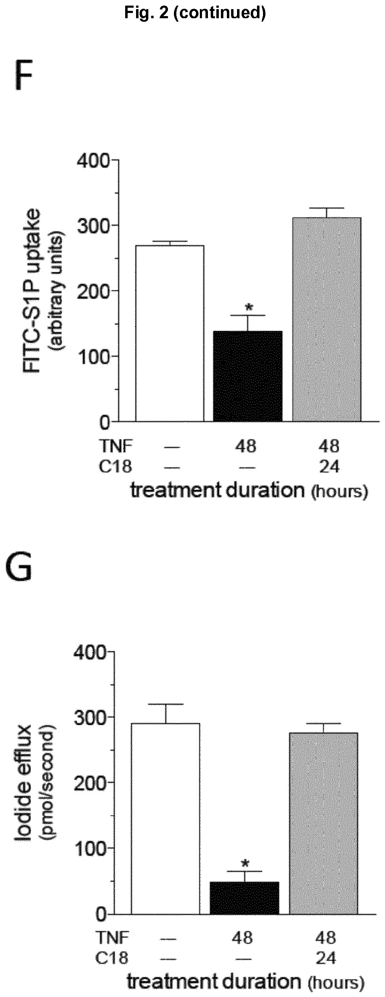

**Figure 2****. C18 increases wild-type CFTR protein expression and function by a proteostatic mechanism**
   **(A)** Cerebral arteries isolated from naïve mice treated with C18 (3 mg/kg daily for 2 days; n=5) have higher cystic fibrosis transmembrane conductance regulator (CFTR) protein expression than arteries collected from vehicle controls (n=5). **(B)** C18 does not influence cerebral artery CFTR mRNA expression (Con n=6, C18 n=5). **(C)** C18 (6 µmol/L; 24 h) increases CFTR protein expression in baby hamster kidney fibroblast cells stably expressing human CFTR (n=12 for both groups); **(D)** C18 does not influence CFTR mRNA expression in this system (n=6 for both groups). **(E)** Tumor necrosis factor (TNF; 10 ng/ml for 48 h) down-regulates CFTR protein expression in mesenteric artery primary vascular smooth muscle cells (control: n=8; TNF: n=8); co-incubating TNF with C18 (6 µmol/L; 24 h) following 24 h TNF incubation (i.e., 48 h TNF + 24 h C18) fully restores CFTR protein expression (n=7). The restoration of CFTR protein expression in vascular smooth muscle cells correlates with the normalization of attenuated **(F)** FITC-labeled sphingosine-1-phosphate uptake (measured as an increase in fluorescence intensity at 525 nm by a standard fluorescence-activated cell sorting analysis technique; control: n=7; TNF: n=10; TNF+C18: n=6) and **(G)** forskolin-stimulated iodide efflux (control: n=7; TNF: n=6; TNF+C18: n=6). All data are mean ± s.e.m. In *Panels A to D,* * denotes P<0.05 for an unpaired comparison with a Mann-Whitney test; in *Panels E to* G, * denotes P<0.05 for unpaired comparisons to the control with a Kruskal-Wallis test and Dunn's *post-hoc* test. Abbreviations: Con - control; CFTR - cystic fibrosis transmembrane conductance regulator; TNF - tumor necrosis factor.

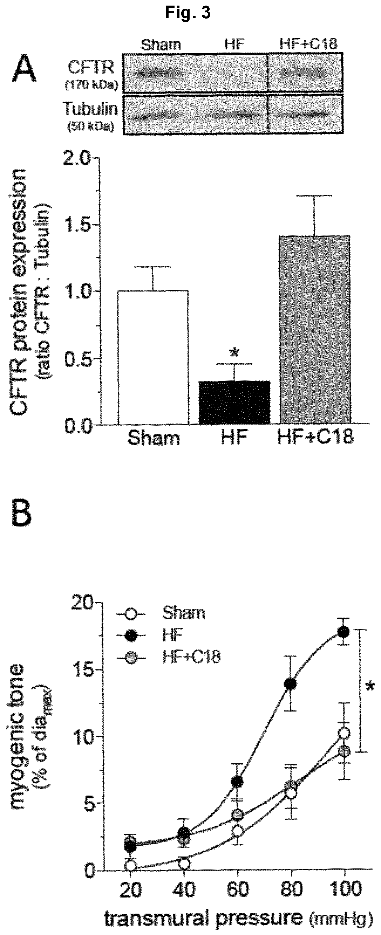

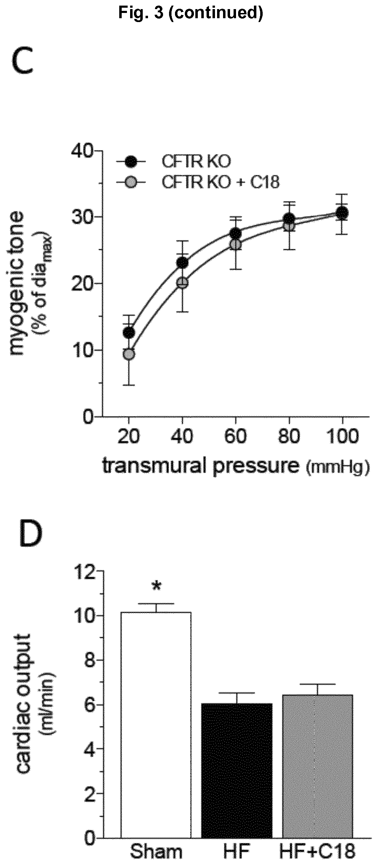

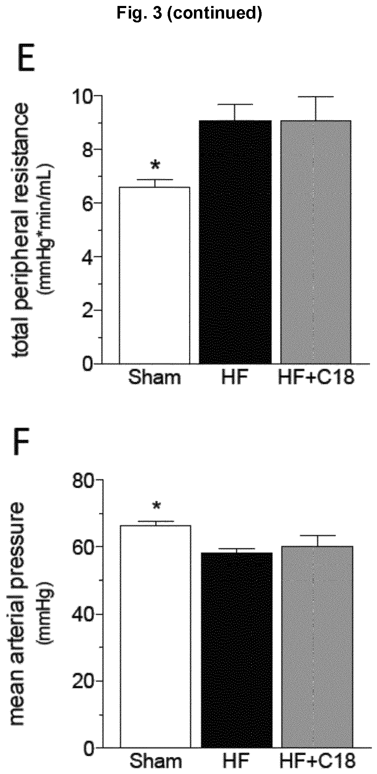

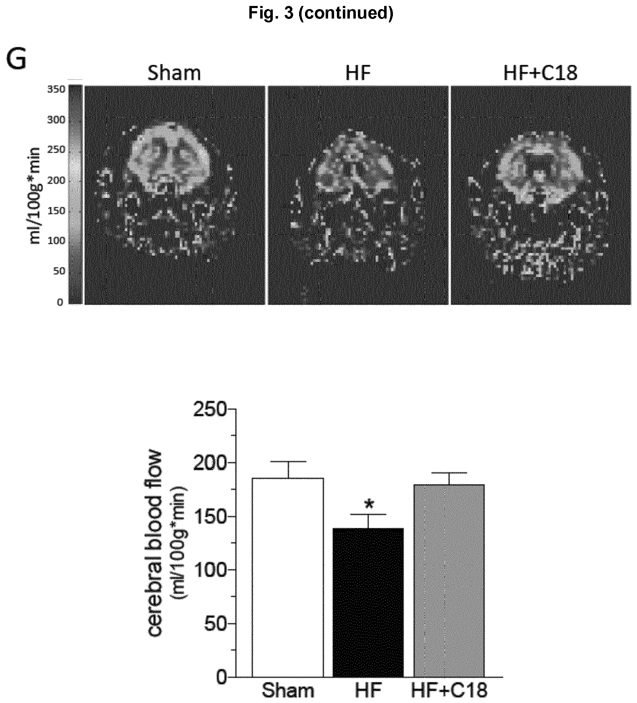

**Figure 3****. C18 restores cerebral perfusion in heart failure**
   **(A)** Cerebral arteries isolated from mice with heart failure (HF; 6 weeks post-left anterior descending coronary artery ligation) have reduced cystic fibrosis transmembrane conductance regulator (CFTR) protein expression (n=5), relative to arteries isolated from sham-operated controls (n=6). C18 treatment *in vivo* (3 mg/kg daily for 2 days) eliminates this reduction in cerebral artery CFTR protein expression (n=8). **(B)** C18 treatment *in vivo* reduces myogenic tone in posterior cerebral arteries isolated from HF mice, an effect **(C)** not observed in posterior cerebral arteries isolated from CFTR knockout (KO) mice. Mean maximal vessel diameters at 45 mmHg (diaₘₐₓ) are: Sham=146±9 µm, n=5 from 3 mice; HF=149±8 µm, n=6 from 4 mice; HF+C18=155±8 µm, n=8 from 6 mice (Kruskal-Wallis test P=N.S. for diaₘₐₓ); and CFTR KO=142±5 µm, n=6 from 3 mice; CFTR KO+C18=145±5 µm, n=6 from 3 mice (Mann-Whitney test P=N.S. for diaₘₐₓ). Relative to sham-operated controls, mice with HF have **(D)** reduced cardiac output (sham: n=6; HF: n=8; HF+C18: n=8), **(E)** elevated total peripheral resistance (n=6 for all groups) and **(F)** reduced mean arterial pressure (n=6 for all groups). **(G)** Shown are representative Magnetic Resonance Imaging perfusion maps that were used to determine forebrain cortical cerebral blood flow. HF stimulates a reduction in cerebral perfusion; C18 treatment significantly improves cerebral perfusion in mice with HF (sham: n=6; HF: n=8; HF+C18: n=8). All data are mean ± s.e.m. For *Panels A and G*, * denotes P<0.05 for unpaired comparisons to the sham with a Kruskal-Wallis test and Dunn's *post-hoc* test; for *Panel B,* * denotes P<0.05 for unpaired comparisons to sham with a two-way ANOVA and Tukey's *post-hoc* test; in *Panel C*, groups were statistically compared with a two-way ANOVA (P=N.S.); and in *Panels E to G*, * denotes P<0.05 for unpaired comparisons to HF with a Kruskal-Wallis test and Dunn's *post-hoc* test. All samples in the representative western blot image displayed in *Panel A* originate from the same film, but the HF+C18 sample was not adjacently positioned on this film, as shown in this figure. Abbreviations: Con - control; CFTR - cystic fibrosis transmembrane conductance regulator; diaₘₐₓ - maximal vessel diameter; HF - heart failure; KO - knockout.

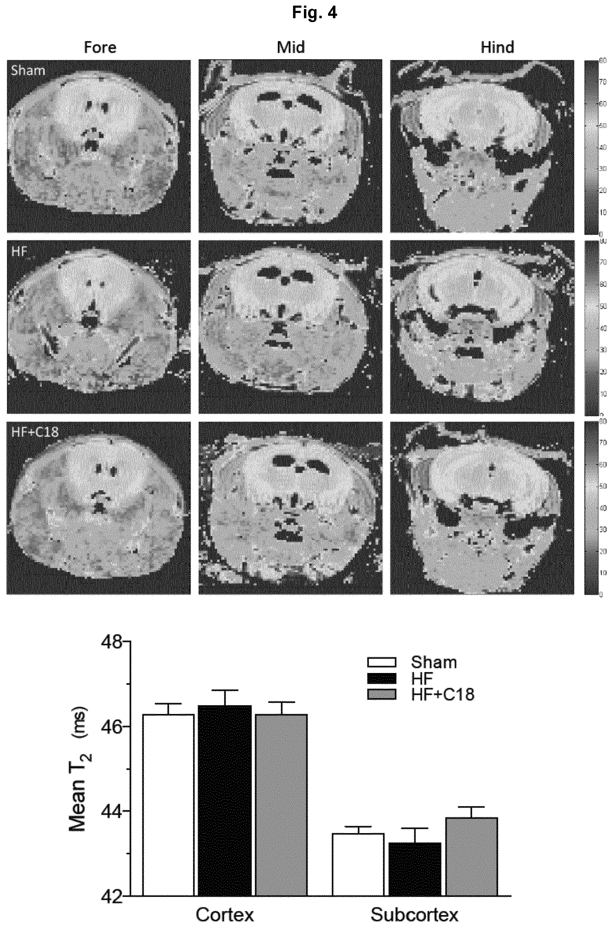

**Figure 4****. C18 does not induce cerebral edema**
   Shown are representative quantitative T₂ maps that assess brain water content as T₂ relaxation times in Magnetic Resonance images. A total of nine T₂ maps were assessed per mouse, which cover the fore-, mid- and hind-brain regions. The representative images display slices from the fore- (*left*), mid- (*center*) and hind-brain (*right*) regions of sham (*top*), heart failure (HF; *middle*) and C18-treated (3 mg/kg daily for 2 days) HF mice (*bottom*)*.* Neither HF nor C18 treatment in HF mice induces an alteration in the T₂ relaxation times in any region of interest within any of the slices assessed. The data were therefore pooled to yield a mean T₂ relaxation time for the cortical and sub-cortical region and graphically presented (sham: n=7; HF: n=7; HF+C18: n=6; Mann-Whitney P=N.S. between groups in the cortical and sub-cortical regions, respectively). All data are mean ± s.e.m. Groups were statistically compared with a Kruskal-Wallis test (P=N.S.). Abbreviations: HF - heart failure.

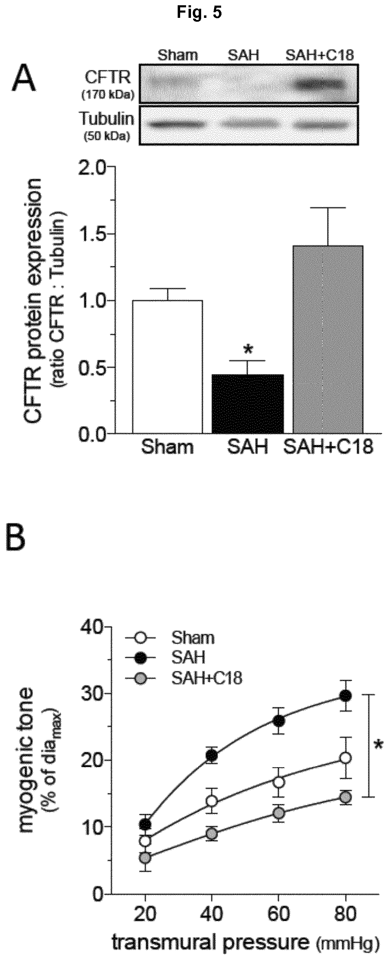

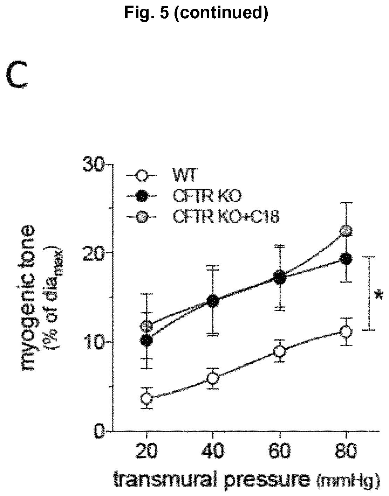

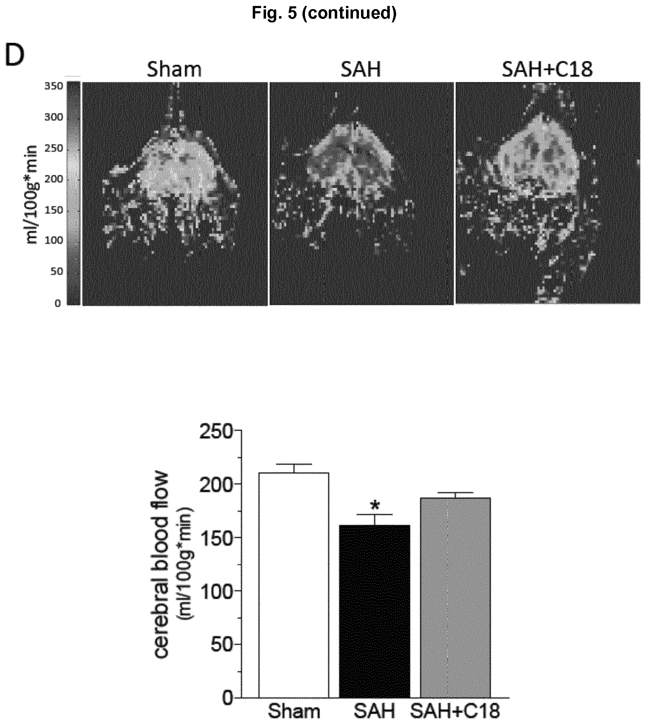

**Figure 5****. C18 restores cerebral perfusion in subarachnoid hemorrhage**
   **(A)** Cerebral arteries isolated from mice with subarachnoid hemorrhage (SAH; 2 days post-SAH induction) have reduced cystic fibrosis transmembrane conductance regulator (CFTR) protein expression (n=6), relative to arteries isolated from sham-operated controls (n=6). C18 treatment *in vivo* (3 mg/kg daily for 2 days) eliminates this reduction in artery CFTR protein expression (n=6). **(B)** C18 treatment *in vivo* reduces myogenic tone in olfactory arteries isolated from mice with SAH, an effect **(C)** not observed in olfactory arteries isolated from CFTR knockout (KO) mice. Mean maximal vessel diameters at 45 mmHg (diaₘₐₓ) are: Sham=113±3 µm, n=5 from 3 mice; SAH=109±6 µm, n=6 from 6 mice; SAH+C18=104±12 µm, n=5 from 4 mice (Kruskal-Wallis test P=N.S. for diaₘₐₓ); and CFTR WT=98±6 µm, n=8 from 4 mice; CFTR KO=110±8 µm, n=5 from 4 mice; CFTR KO+C18=96±6 µm, n=6 from 3 mice (Kruskal-Wallis test P=N.S. for diaₘₐₓ). **(D)** Shown are representative Magnetic Resonance Imaging perfusion maps that were used to determine forebrain cortical cerebral blood flow. SAH stimulates a reduction in cerebral perfusion; C18 treatment significantly improves cerebral perfusion in mice with SAH (sham: n=10; SAH: n=5; SAH+C18: n=9). All data are mean ± s.e.m. In *Panels A and D,* * denotes P<0.05 for unpaired comparisons to the SAH with a Kruskal-Wallis test and Dunn's *post-hoc* test; In *Panel B,* * denotes P<0.05 for unpaired comparisons to SAH with a two-way ANOVA and Tukey's *post-hoc* test; In *Panel C*, Groups were statistically compared with a two-way ANOVA (P=N.S.). Abbreviations: CFTR - cystic fibrosis transmembrane conductance regulator; diaₘₐₓ - maximal vessel diameter; HF - heart failure; KO - knockout; SAH - subarachnoid hemorrhage.

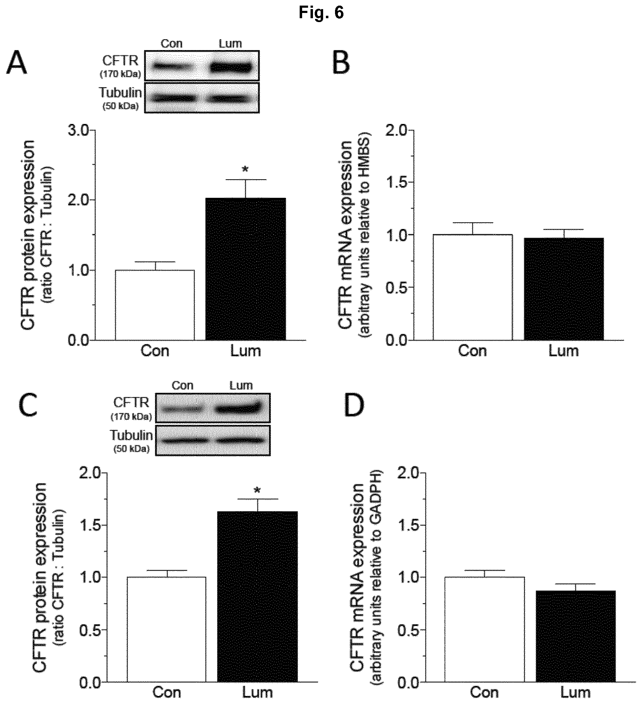

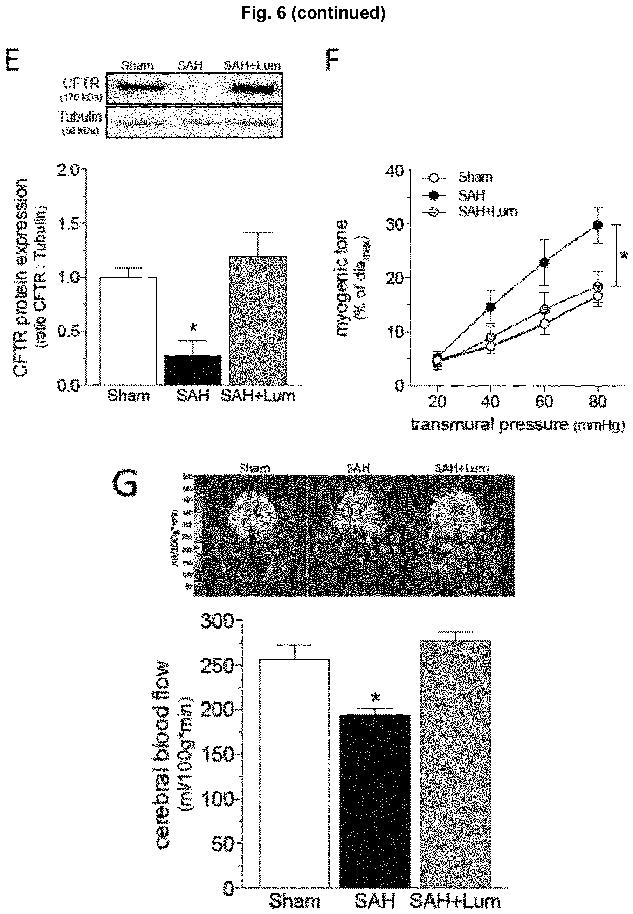

**Figure 6****: Lumacaftor increases wild-type CFTR protein expression by a proteostatic mechanism and restores cerebral perfusion in subarachnoid hemorrhage**
   **(A)** Cerebral arteries isolated from naïve mice treated with lumacaftor (Lum; 3 mg/kg daily for 2 days; n=6) have higher cystic fibrosis transmembrane conductance regulator (CFTR) protein expression than arteries collected from vehicle controls (n=7). **(B)** lumacaftor does not influence cerebral artery CFTR mRNA expression (n=5 for both groups). **(C)** lumacaftor (6 µmol/L; 24 h) increases CFTR protein expression in baby hamster kidney fibroblast cells stably expressing human CFTR (n=13 for both groups); **(D)** lumacaftor does not influence CFTR mRNA expression in this system (n=6 for both groups). **(E)** Cerebral arteries isolated from mice with subarachnoid hemorrhage (SAH; 2 days post-SAH induction) have reduced CFTR protein expression (n=5), relative to arteries isolated from sham-operated controls (n=6). lumacaftor treatment *in vivo* (3 mg/kg daily for 2 days) eliminates this reduction in cerebral artery CFTR protein expression (n=6). **(F)** lumacaftor treatment *in vivo* reduces myogenic tone in olfactory arteries isolated from mice with SAH. Mean maximal vessel diameters at 45 mmHg (diaₘₐₓ) are: Sham=91±3 µm, n=7 from 4 mice SAH=86±2 µm, n=6 from 3 mice; SAH+Lum=87±4 µm, n=7 from 4 mice (Kruskal-Wallis test P=N.S. for diaₘₐₓ). **(G)** Shown are representative Magnetic Resonance Imaging perfusion maps that were used to determine the cerebral blood flow. SAH stimulates a reduction in cerebral perfusion; lumacaftor treatment significantly improves cerebral perfusion in mice with SAH (sham: n=6; SAH: n=5; SAH+Lum: n=6). All data are mean ± s.e.m. In *Panels A to D,* * denotes P<0.05 for an unpaired comparison with a Mann-Whitney test; in *Panels E to G,* * denotes P<0.05 for unpaired comparisons to SAH with a Kruskal-Wallis test and Dunn's *post-hoc* test. In *Panel F,* * denotes P<0.05 for unpaired comparisons to SAH with a two-way ANOVA and Tukey's *post-hoc* test. Abbreviations: CFTR - cystic fibrosis transmembrane conductance regulator; diaₘₐₓ - maximal vessel diameter; Lum - lumacaftor; SAH - subarachnoid hemorrhage.

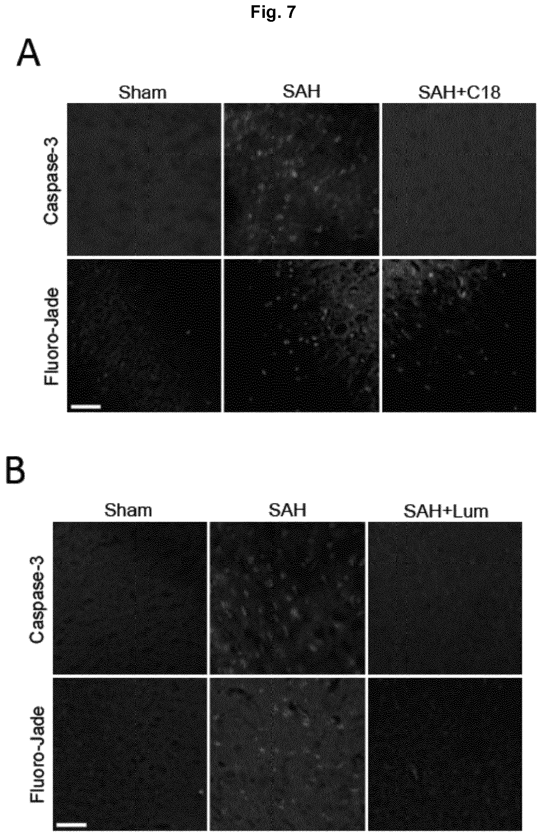

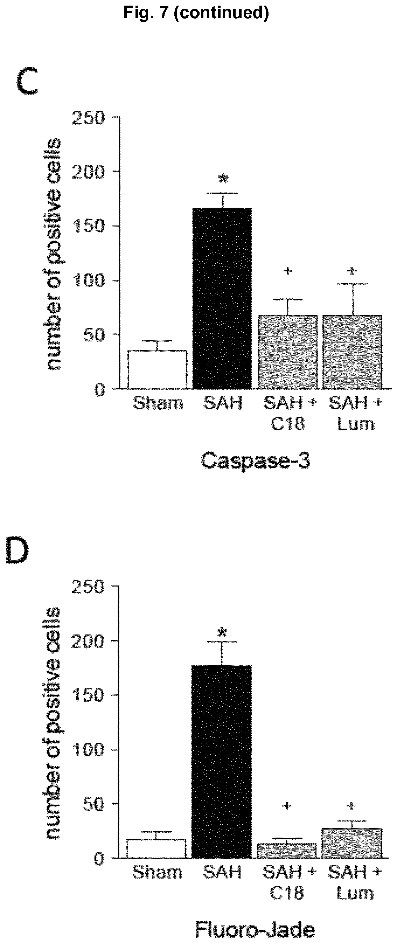

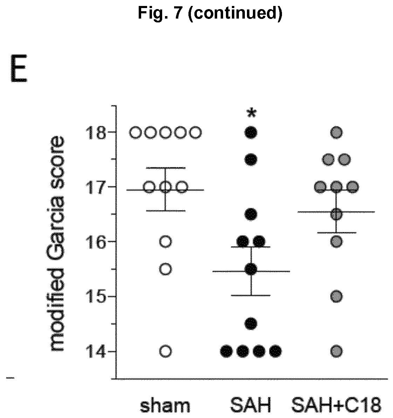

**Figure 7****: CFTR correction reduces neuronal injury in subarachnoid hemorrhage**
   Shown are representative images of cortical cells stained for cleaved caspase-3 expression (top) and with Fluoro-Jade (bottom) for cohorts involving **(A)** C18 (3 mg/kg daily for 2 days) and **(B)** lumacaftor (Lum; 3 mg/kg daily for 2 days) treatment regimes. Subarachnoid hemorrhage (SAH; n=13 mice) increases the number of **(C)** caspase-3 and **(D)** Fluoro-Jade positive cells relative to sham-operated controls (n=12); both C18 (n=6) and lumacaftor (n=5) abolish the increase in caspase-3 and Fluoro-Jade staining. **(E)** Mice with SAH (n=11) have a lower Modified Garcia score (maximum score = 18; blinded assessments made 2 days post-SAH), compared to sham-operated mice (n=11); C18 treatment (n=10) restores the neurological score to the sham level. All data are mean ± s.e.m. * denotes P<0.05 for a unpaired comparisons to sham with a Kruskal-Wallis test and Dunn's *post-hoc* test. In Panels *C and D,* + denotes P<0.05 compared to untreated SAH with a Kruskal-Wallis test and Dunn's *post-hoc* test. Abbreviations: Lum - lumacaftor; SAH - subarachnoid hemorrhage.

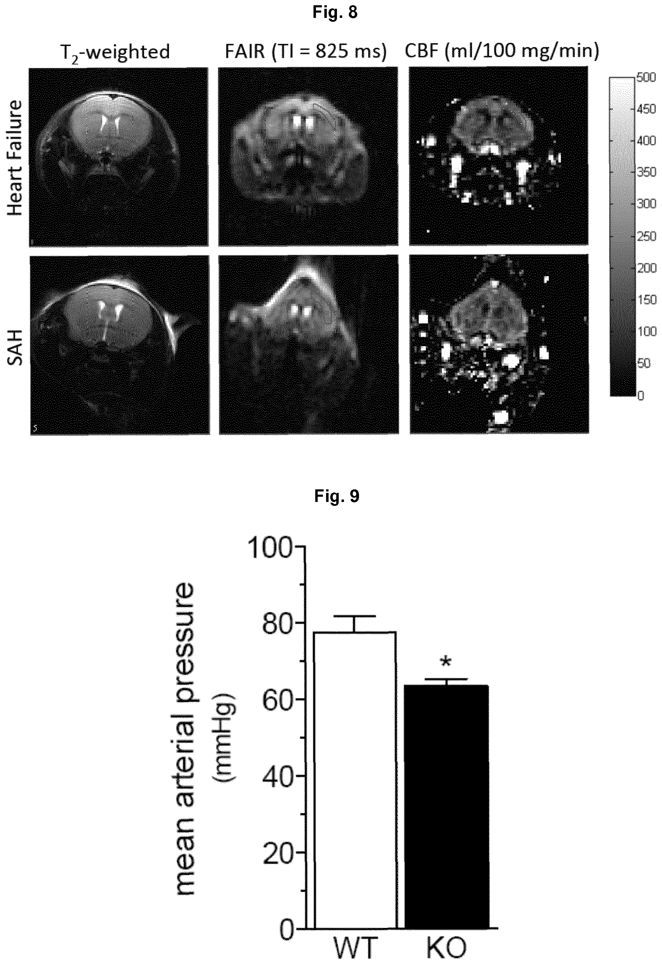

**Figure 8****: Region of interest placement in FAIR-EPI and analysis images.**
   Shown are representative anatomical T2-weighted, flow-sensitive alternating inversion recovery (FAIR) T1-weighted, and FAIR cerebral blood flow (CBF) maps for mice that underwent either the heart failure (top row) or subarachnoid hemorrhage (SAH; bottom row) surgical procedure. Regions of interest were manually drawn on forebrain slice FAIR images acquired at an inversion time (TI) of 825 ms using MIPAV software. The regions of interest encompass approximately 1 mm3 of the cortical volume within 1 hemisphere, as shown. The regions of interest were then copied directly onto the CBF maps derived from FAIR images at the individual inversion times. SAH mice and shamoperated controls displayed varying degrees of echo-planar imaging (EPI) distortion (best visualized in the FAIR image); EPI-distortion was minimal in heart failure mice.
   Abbreviations: CBF - cerebral blood flow; EPI - echo-planar imaging; FAIR - flow-sensitive alternating inversion recovery; SAH - subarachnoid hemorrhage.
**Figure 9****: Mean arterial pressure in CFTR knockout mice**
   Mean arterial pressure is lower in cystic fibrosis transmembrane conductance regulator knockout mice (CFTR KO; CFTRtm1Unc; n=6), relative to wild-type littermates (WT; n=6). * denotes P<0.05 for an unpaired t-test comparison.
   Abbreviations: CFTR - cystic fibrosis transmembrane conductance regulator; KO - knockout; WT - wild-type
**Figure 10****: Phenylephrine responses in mouse cremaster** arteries
   Phenylephrine stimulates dose-dependent vasoconstriction in cremaster skeletal muscle arteries isolated from wild-type and cystic fibrosis transmembrane conductance regulator knockout mice (CFTR KO; CFTRtm1Unc) mice. The phenylephrine dose-response relationship is not altered by **(A)** CFTR inhibition
   *in vitro* (100 nmol/L CFTR(inh)-172 for 30 minutes) or **(B)** CFTR gene deletion. Mean maximal vessel diameters at 60 mmHg (diamax) are: wild-type (*Panel A*): 72±3 µm, n=5 from 4 mice; CFTR KO (*Panel B*)*:* 88±4 µm, n=6 from 4 mice; and wild-type littermates (*Panel B*)*:* 79±4 µm, n=5 from 2 mice. Dose-response curves in Panel A are compared with a paired two-way ANOVA (P=NS); curves in Panel B are compared with an unpaired two-way ANOVA (P=NS). For *Panel B,* KO and wild-type diamax values are not different (t-test P=NS).
   Abbreviations: CFTR - cystic fibrosis transmembrane conductance regulator; diamax - maximal vessel diameter; KO - knockout; NS - not significant.

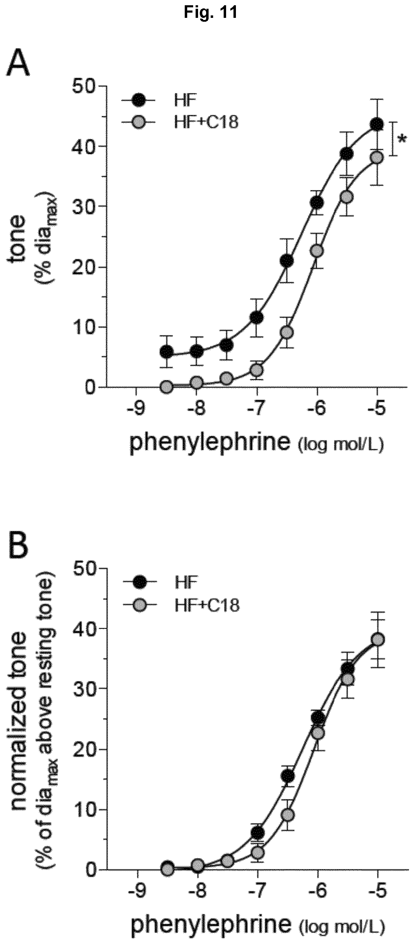

**Figure 11****: Phenylephrine responses in mouse posterior cerebral arteries following in vivo C18 treatment**
   **(A)** Phenylephrine stimulates dose-dependent vasoconstriction in posterior cerebral arteries isolated from mice with heart failure (HF) and mice with heart failure treated with C18 *in vivo* (3 mg/kg *i.p.* daily for 2 days). Statistical analysis identifies a significant difference between the two curves (i.e., there is significantly higher tone in arteries from HF, relative to HF+C18). However, **(B)** when the data are normalized to the basal tone (toneactive - tonerest, where toneactive is the tone at given phenylephrine concentration and tonerest is the tone immediately prior to stimulation), the dose-response relationships are not significantly different. Mean maximal vessel diameters at 45 mmHg (diamax) are: HF: 137±7 µm, n=5 from 3 mice and HF+C18: 140±8 µm, n=5 from 4 mice (t-test P=NS). In both panels, * denotes P<0.05 with a two-way ANOVA.
   Abbreviations: diamax - maximal vessel diameter; HF - heart failure; NS - not significant.

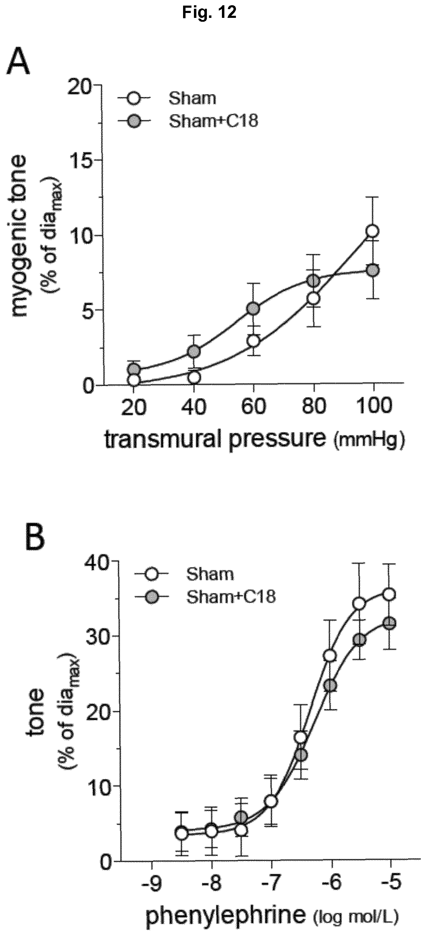

**Figure 12****: *In vivo* C18 treatment does not alter myogenic tone or phenylephrine responses in posterior cerebral arteries isolated from sham-operated mice**
   *In vivo* C18 treatment (3 mg/kg *i.p.* daily for 2 days) does not alter **(A)** myogenic tone or **(B)** phenylephrine responses in posterior cerebral arteries isolated from sham-operated mice. Mean maximal vessel diameters at 45 mmHg (diamax) in *Panel A:* Sham: 146±9 µm, n=5 from 3 mice and Sham+C18: 138±9 µm, n=7 from 4 mice (t-test P=NS); and in *Panel B:* Sham: 148±7 µm, n=6 from 3 mice and Sham+C18: 130±10 µm, n=5 from 3 mice (t-test P=NS). Curves in both panels are compared with a two-way ANOVA (P=NS). Abbreviations: diamax - maximal vessel diameter; NS - not significant.

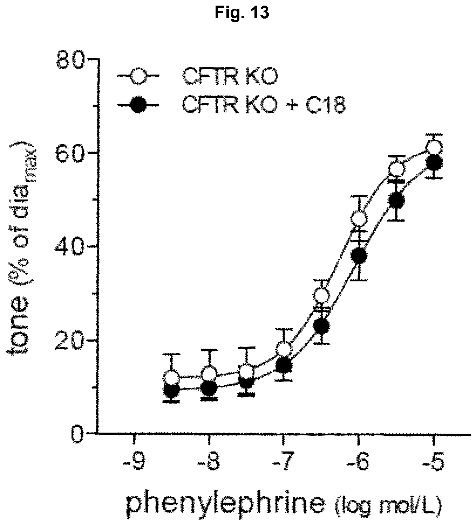

**Figure 13****: Phenylephrine responses in posterior cerebral arteries from CFTR knockout mice treated with C18**
   Phenylephrine stimulates dose-dependent vasoconstriction in posterior cerebral arteries isolated from cystic fibrosis transmembrane conductance regulator knockout mice (CFTR KO; CFTRtm1Unc). *In vivo* C18 treatment (3 mg/kg *i.p.* daily for 2 days) does not alter the phenylephrine dose-response
   relationship. Mean maximal vessel diameters at 45 mmHg (diamax) are: CFTR KO: 142±5 µm, n=6 from 3 mice and CFTR KO + C18: 145±5 µm, n=6 from 3 mice (t-test P=NS). Curves are compared with a two-way ANOVA (P=NS).
   Abbreviations: CFTR - cystic fibrosis transmembrane conductance regulator; diamax - maximal vessel diameter; KO - knockout; NS - not significant.

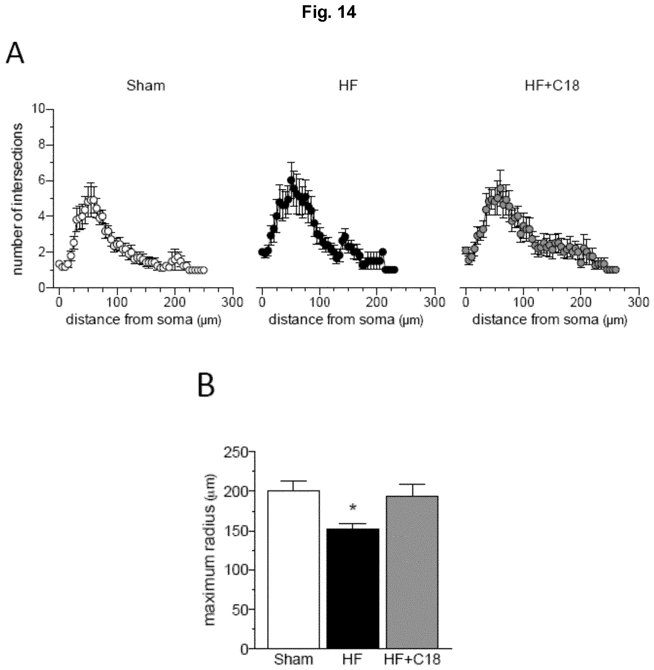

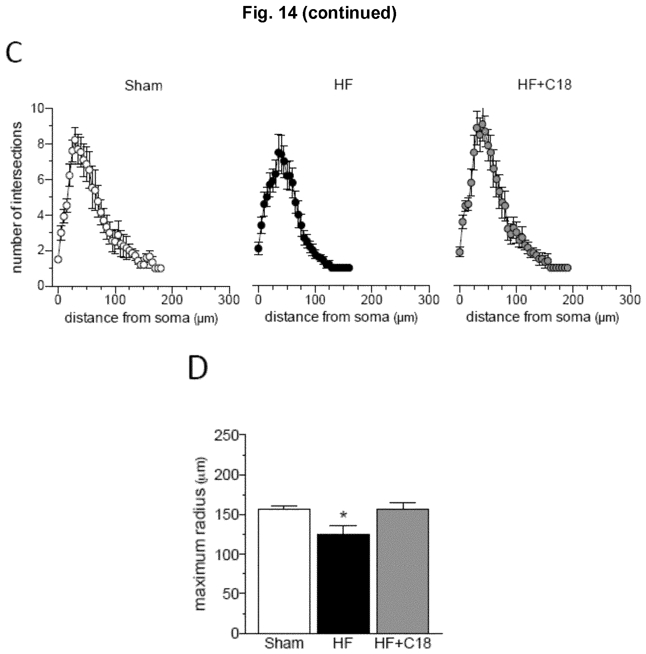

**Figure 14****: Breakdown Sholl analysis of apical and basal dendrites**
   **(A)** Shown are Sholl analysis histograms plotting the number of dendrite intersections (i.e., dendritic branching) versus dendritic length (i.e., distance from neuronal soma) for ***apical** dendrites.* No differences in branching morphology are observed across the sham (n=13 neurons fromN=4 mice), heart failure (HF; n=11; N=4) and HF+C18 (3 mg/kg *i.p.* daily for 2 weeks; n=11; N=4) groups; however, apical dendritic length is shorter in HF mice, relative to the sham and HF+C18 groups. Accordingly, **(B)** mean apical dendrite length (i.e., maximum radius) is significantly reduced in HF mice relative to sham mice; this effect is normalized by C18 treatment. Similarly, **(C)** Sholl analysis histograms for ***basal dendrites*** show no differences in branching morphology across the sham (n=12; N=4), HF (n=13; N=4) and HF+C18 (n=11; N=4) groups; however, basal dendritic length is shorter in HF mice, relative to the sham and HF+C18 groups. Accordingly, **(D)** mean basal dendrite length (i.e., maximum radius) is significantly reduced in HF mice relative to sham mice, an effect that is normalized by C18 treatment. * denotes P<0.05 for unpaired comparisons to the sham with a one-way ANOVA and Dunnett's *post-hoc* test.
   Abbreviations: HF - heart failure.

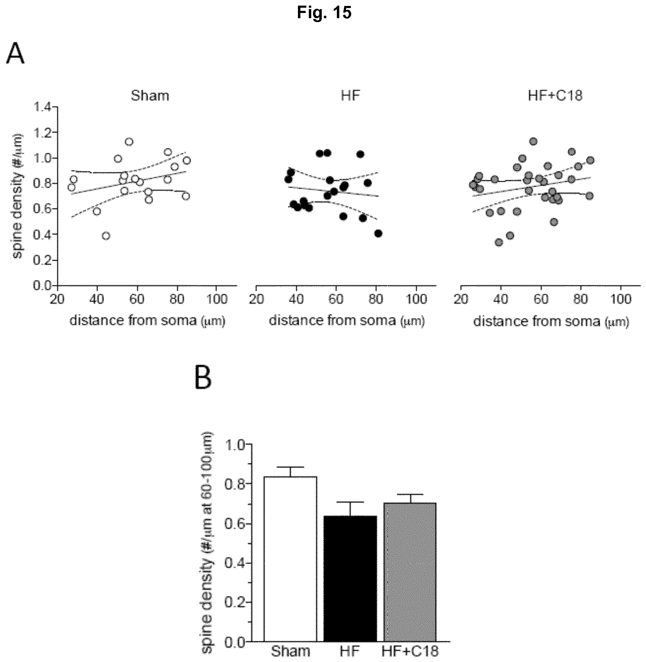

**Figure 15****: Spine density analysis of apical dendrites**
   **(A)** In sham (n=8 neurons from N=4 mice), heart failure (HF; n=6; N=3) and HF+C18 mice (3 mg/kg *i.p.* daily for 2 weeks; n=10; N=4), ***apical dendrite*** spine density (i.e., a measure of synapse density) is relatively consistent over the length of the dendrite, yielding slopes that are not statistically different from zero. **(B)** Although tendencies are present, mean apical dendrite spine density measures at 60-100µm from soma does not reach statistical significance. The groups were statistically compared with a one-way ANOVA (P=NS). Abbreviations: HF - heart failure; NS - not significant.

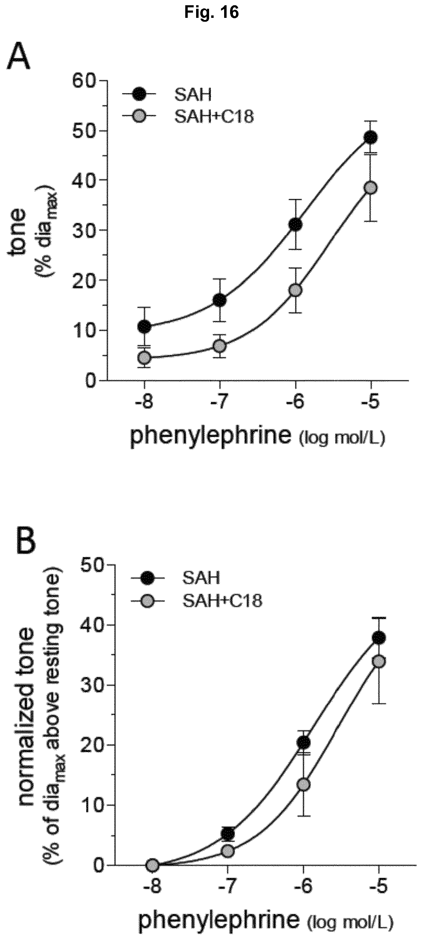

**Figure 16****: Phenylephrine responses in mouse olfactory cerebral arteries following in vivo C18 treatment**
   **(A)** Phenylephrine stimulates dose-dependent vasoconstriction in olfactory cerebral arteries isolated from mice with subarachnoid hemorrhage (SAH) and SAH mice treated with C18 *in vivo* (3 mg/kg *i.p.* daily for 2 days). Although there is a clear tendency for separation, the two curves are not statistically different. **(B)** When the data are normalized to the basal tone (toneactive - tonerest, where toneactive is the tone at given phenylephrine concentration and tonerest is the tone immediately prior to stimulation), the separation of the dose-response relationships is minimal. Mean maximal vessel diameters at 45 mmHg (diamax) are: SAH: 109±6 µm, n=6 from 6 mice and SAH+C18: 105±12 µm, n=5 from 4 mice (t-test P=NS). Curves are compared with a two-way ANOVA.
   Abbreviations: diamax - maximal vessel diameter; NS - not significant; SAH - subarachnoid hemorrhage.

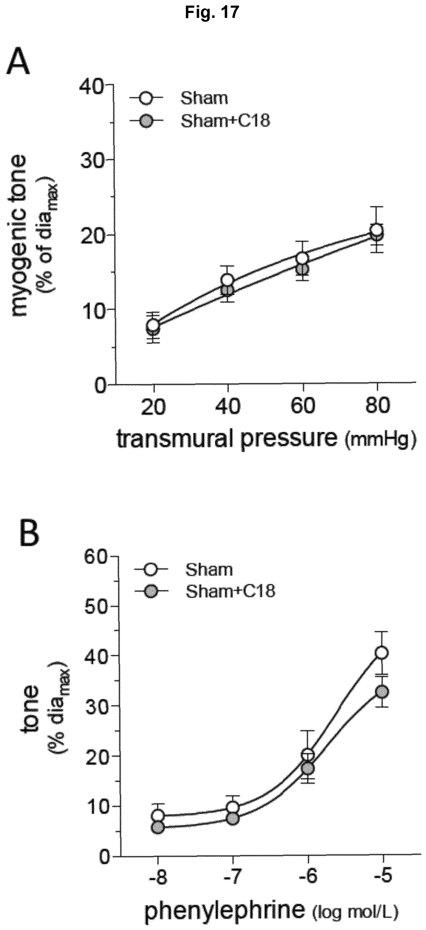

**Figure 17****: C18 does not impact myogenic tone or phenylephrine responses in sham-operated mice**
   *In vivo* C18 treatment (3 mg/kg *i.p.* daily for 2 days) does not alter **(A)** myogenic tone or **(B)** the phenylephrine dose-response relationship in olfactory cerebral arteries isolated from sham-operated mice. Mean maximal vessel diameters at 45 mmHg (diamax) are: Sham: 113±3 µm, n=5 from 3 mice; and Sham+C18: 110±8 µm, n=6 from 4 mice (t-test P=NS). Curves are compared with a two-way ANOVA.
   Abbreviations: diamax - maximal vessel diameter; NS - not significant.

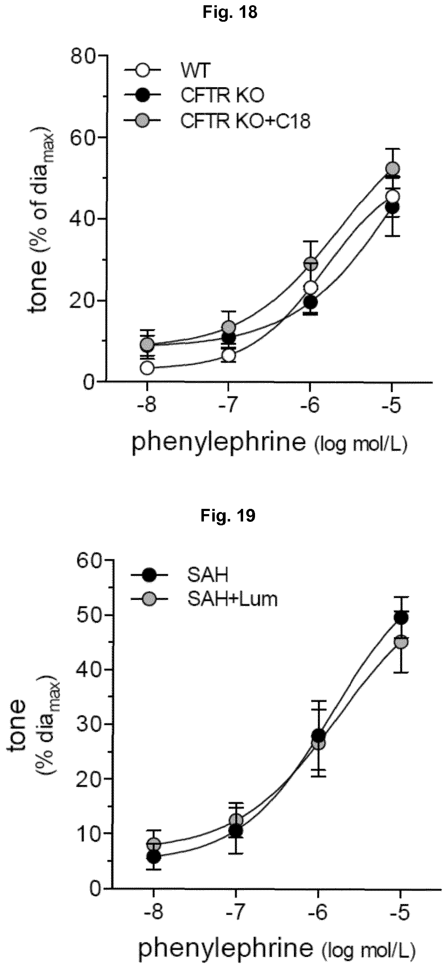

**Figure 18****: Phenylephrine responses in olfactory cerebral arteries isolated from CFTR knockout mice**
   Phenylephrine stimulates dose-dependent vasoconstriction in olfactory cerebral arteries isolated from cystic fibrosis transmembrane conductance regulator knockout mice (CFTR KO; CFTRtm1Unc) that is similar to that of wild-type (WT) littermates. *In vivo* C18 treatment (3 mg/kg *i.p.* daily for 2 days) does not alter the phenylephrine dose-response relationship. Mean maximal vessel diameters at 45 mmHg (diamax) are: WT: 98±6 µm, n=8 from 4 mice; CFTR KO: 110±8 µm, n=5 from 4 mice and CFTR KO + C18: 96±6 µm, n=6 from 3 mice (one-way ANOVA P=NS). Curves are compared with a two-way ANOVA.
   Abbreviations: CFTR - cystic fibrosis transmembrane conductance regulator; diamax - maximal vessel diameter; KO - knockout; NS - not significant; WT - wild-type.
**Figure 19****. Phenylephrine responses in mouse olfactory cerebral arteries following in vivo lumacaftor treatment**
   *In vivo* lumacaftor (Lum) treatment (3 mg/kg *i.p.* daily for 2 days) does not alter the phenylephrine dose-response relationship in olfactory cerebral arteries isolated from mice with subarachnoid hemorrhage (SAH). Mean maximal vessel diameters at 45 mmHg (diamax) are: SAH: 86±2 µm, n=6 from 3 mice; and SAH+Lum: 87±4 µm, n=7 from 4 mice (t-test P=NS). Curves are compared with a two-way ANOVA.
   Abbreviations: diamax - maximal vessel diameter; Lum - lumacaftor; NS - not significant; SAH - subarachnoid hemorrhage.

The present invention is further illustrated by the following non-limiting example.

### EXAMPLE - (those parts of the example relating to C18 are not part of the invention)

### METHODS AND REAGENTS

### Reagents

The anti-human cystic fibrosis transmembrane conductance regulator (CFTR) antibody used to detect CFTR in baby hamster kidney fibroblast cells ("antibody 596") the CFTR corrector therapeutic "C18" (CF-106951; see WO 2007/021982; 1-(benzo[d][1,3]dioxol-5-yl)-N-(5-((2-chlorophenyl)(3-hydroxypyrrolidin-1-yl)methyl)thiazol-2-yl)cyclopropanecarboxamide) were acquired through the *Cystic Fibrosis Foundation Therapeutics* Chemical and Antibody Distribution Programs
(http://www.cff.org/research). Dr. John Riordan (University of North Carolina - Chapel Hill) provided the "596 CFTR antibody" and Dr. Robert Bridges (Rosalind Franklin University of Medicine and Science, Chicago, USA) provided the C18 compound. The 596 CFTR antibody targets amino acids 1204-1211 (i.e., located in nucleotide binding domain 2) (1). Fluorescein-labeled S1P (FITC-S1P) was purchased from Echelon Biosciences (via Cedarlane Laboratories; Burlington, Canada); lumacaftor was purchased from Selleck Chemicals (Cedarlane Laboratories); recombinant tumor necrosis factor was purchased from Sigma-Aldrich Canada (Oakville, Canada; cat# T6674); and protease inhibitor cocktail tablets (Complete^{®}; used in western
blot lysis buffers) were purchased from Roche (Mississauga, Canada). All other chemical reagents were purchased from Sigma-Aldrich. MOPS-buffered salt solution contained [mmol/L]: NaCl 145, KCI 4.7, CaCl2 3.0, MgSO4 •7H2O 1.17, NaH2PO4 •2H2O 1.2, pyruvate 2.0, ethylenediaminetetraacetic acid (EDTA) 0.02, 3-morpholinopropanesulfonic acid (MOPS) 3.0, and glucose 5.0.

### CFTR Mutant and Knockout Mice

Male mice homozygous for the ΔF508 CFTR mutation (CFTRtm1EUR; designated "ΔF508" in the present study) (2), CFTR gene deletion (CFTRtm1 Unc; designated CFTR-/-) and the complimentary wildtype control littermates were obtained from an established colony at the *Hospital for Sick Children,* Toronto (all CFTR-/-, CFTRΔF508 and wild-type littermates are mixed strains). It was found that CFTR-/- mice were highly prone to dying under anesthesia. While this mortality was not an issue for experiments involving cerebral artery isolation (e.g., Figures 3C and 5C), it significantly confounded cerebral blood flow and systemic hemodynamic measurements. Unlike CFTR-/- mice, CFTRΔF508 mutant mice were not nearly as prone to dying during anesthesia. Since the CFTRΔF508 mutation profoundly reduces CFTR trafficking to the plasma membrane (3), CFTR activity is very low in the CFTRΔF508 mouse model (2,4,5). Thus, the CFTRΔF508 model provided an adequate alternative to CFTR-/-mice for cerebral blood flow and systemic hemodynamic measurements. We did not pursue the underlying cause of the CFTR-/- mortality. Both CFTR-/- and CFTRΔF508 are widely known to adversely react to stress. As examples, housing conditions and transport are two notable stressors that can significantly increase mortality (6). Interestingly, CFTRΔF508 mice display a less severe phenotype than CFTR-/- mice (5,6): this is presumed to be due to the small amount of residual CFTR activity that is present in CFTRΔF508 animals (2). In this context, we suspect that the CFTR-/- mice may have been more sensitive to external environmental stressors in our animal facility (e.g., housing conditions, noise and
handling), which ultimately manifested in heightened mortality under anesthesia. As an experimental model, CFTRΔF508 mice possess several abnormalities associated with the loss of CFTR function. Intestinal complications are the most pronounced pathological effect of the CFTR mutation: this also represents the primary cause of post-natal mortality (6,7). Common gastrointestinal issues include thick mucus retention, dysmotility and a strong propensity for bowel obstruction. CFTRΔF508 mice are 40-50% smaller in weight/size relative to wild-type counterparts, although they thrive well into adulthood (6,7). Several other differences relative to wild-type mice have been observed; however, these appear to be relatively minor (6,7). For example, altered chloride currents and/or sodium transport have been noted in certain tissues (e.g., kidney, gall bladder, nasal epithelium), but the pathological significance of these differences is not clear, since the tissues do not appear to be histologically compromised (6,7). Of note, CFTRΔF508 mice (and CFTR mutant mice in general) possess a rather mild cystic fibrosis phenotype in relation to human cystic fibrosis. The lower respiratory tract of CFTRΔF508 mice is essentially normal: there are no lower airway epithelial abnormalities and lung inflammation does not spontaneously develop without challenge (6,7). The pancreas, gall bladder, liver, bile duct, male reproductive tract, lacrimal gland and submandibular glands display no obvious histopathology (5-7). The lack of a severe cystic fibrosis phenotype is partially attributed to the expression of a non-CFTR, calcium-activated chloride channel (CACC) in certain mouse tissues that compensates for the loss of CFTR (7,8).

### Global hemodynamic parameters

As previously described (9-11), echocardiographic measurements were collected with a 30 MHz mechanical sector transducer (Vevo 770; Visual Sonics, Toronto, Canada) in conjunction with the mean arterial pressure (MAP) measurements (Millar SPR-671 micro-tip mouse pressure catheter; Inter V Medical Inc., Montreal, Canada). The aortic flow velocity-time integral (VTI) was measured using pulse wave Doppler just above the aortic root. The aortic cross-sectional area (CSA) was calculated from the aortic root dimension (ARD): CSA = π(ARD/2)2. Stroke volume (SV), CO and TPR were calculated as: SV = CSA x VTI; CO = SV x HR; TPR = MAP/CO.

### MRI-based measurement of cerebral perfusion

As described previously (10-12), we utilized a flow-sensitive alternating inversion recovery (FAIR) magnetic resonance imaging (MRI) technique (13) to evaluate cerebral blood flow (CBF). During imaging, mice were immobilized with 1.8% isoflurane delivered through a nose cone. To maintain body temperature, the MRI is equipped with embedded tubes that convect water from an external heater-pump. Respiration was monitored using a pneumatic pillow (SA Instruments, Stonybrook, USA); isoflurane levels were adjusted to maintain respiration at approximately 50 breaths/minute.

FAIR images were obtained using a 7 Tesla micro-MRI system (BioSpec 70/30 USR, Bruker BioSpin, Ettlingen, Germany), including the B-GA12 gradient insert, 72 mm inner diameter linear volume resonator for RF transmission, and anteriorly placed head coil for RF reception. FAIR isolates perfusion as an accelerated T1 signal relaxation following slice-selective compared to non-selective inversion preparation, as per the following equation: CBF = I (1/T1,ss - 1/T1,ns) (ml/(100g*min), where **'ss'** and **'ns'** denote slice-selective and non-selective measurements and I is the blood-brain partition coefficient, defined as the ratio between water concentration per g brain tissue and per ml blood. This coefficient is approximately 90 ml/100g in mice (14). The FAIR optimization used in our study was a single-shot echo planar imaging (EPI) technique with preceding adiabatic inversion. Parameters included echo time of 12.5 ms, repetition time of 17 s, 18 inversion times ranging from 25-to-6825 ms in 400 ms increments, 3 mm slice-selective inversion slab, 18x18 mm field-of-view with 72x72 matrix for 250 µm in-plane resolution, 1 mm slice thickness, and 10 min 12 s data acquisition time. Acquisitions were repeated in fore-, mid-, and hind-brain vertical sections, corresponding to anterior, mixed, and posterior circulations. FAIR images were evaluated by manual prescription (MIPAV, NIH, Bethesda, MD; http://mipav.cit.nih.gov) of sub-hemispheric regions-of-interest (ROls), termed 'global', and local ROls corresponding to cortical and sub-cortical parenchyma in forebrain sections; cortical and paraventricular parenchyma in middle sections; and cortical and midbrain parenchyma in hindbrain sections. ROls were drawn directly on T1-weighted signal images to enable manual correction for intra-scan motion. ROIs were registered with parametric CBF maps to verify absence of bias from high perfusion vessels and meninges. T1 regressions and CBF calculations were performed using Matlab (Mathworks, Natick, MA). All reported CBF values fall within the published range of murine CBF measures for the isoflurane level utilized in this study (15). EPI is particularly prone to magnetic susceptibility-related distortion in the phase-encoding direction (16), which corresponds to the vertical direction in this FAIR protocol. As evidenced in Figure 8, shimming did not sufficiently constrain EPI distortion in the subarachnoid hemorrhage (SAH) surgical model, due to air-tissue boundaries immediately adjacent to the brain at the imaging time-points post-surgery. In certain cases, the distortion was severe enough that the ROIs needed to be placed more laterally in the cortex (Figure 8). However, because the distortion is consistent in all T1-weighted images, this does not bias the T1 measurement, provided the distorted region does not overlap with another region. The relatively consistent ns-T1 values within each cohort (i.e., 7% standard deviation between cohorts, no treatment group differences within cohorts) provide supporting evidence that distortion bias had a negligible impact on the CBF measurements.

### Cell Culture

Procedures for isolating and culturing mesenteric artery smooth muscle cells were carried out as described in (10). Briefly, mesenteric artery segments were isolated, cut into small pieces and digested with trypsin, collagenase and elastase. The resulting cell suspension was washed several times in phosphate-buffered saline and plated in Dulbecco's Modified Eagle Medium (DMEM) culture media containing 10% fetal bovine serum and 1% penicillin-streptomycin. Cell cultures were maintained at 37°C with 5% CO2 and split at 106 cells seeding density. Baby hamster kidney fibroblast cells stably expressing wild-type human CFTR (10,17) were maintained in DMEM/F12 media containing 5% fetal bovine serum and 250 µmol/L methotrexate (which activates the CFTR transgene promoter). Cells were maintained under standard cell culture conditions at 37°C with 5% CO2.

### FACS-based measurement of FITC-S1P uptake

As previously described (10), cell monolayers (treated or untreated) were incubated with 1 µmol/L S1P-FITC for 60 minutes; the cells were then detached by trypsinization, washed twice with ice-cold PBS, filtered through a 35 µm cell strainer and analyzed using the Becton-Dickinson FACS Canto operated by FACS DIVA version 6.1 software. Cell monolayers treated with non-labeled S1P served as background controls. The analysis procedure determined the mean fluorescence intensity (arbitrary units) of each cell population, which is a measure of uptake.

### Iodide Efflux Assessments

Iodide efflux measurements were conducted as previously described (18). Briefly, confluent cell monolayers were loaded with iodide by incubating them in a HEPES-based loading buffer (136 mmol/L Nal, 3 mmol/L KNO3, 2 mmol/L Ca(NO3)2, 11 mmol/L glucose and 20 mmol/L HEPES; pH 7.4) for 1 hour (under standard cell culture conditions of 37oC in 5% CO2). Following iodide loading, cells were washed 4x with iodide-free efflux buffer (where NaNO3 is substituted for Nal). Supernatant iodide levels were quantified with an iodide-selective electrode (Lazar Research Laboratories; Los Angeles, USA), calibrated with NaI standards. Pre-stimulation iodide levels were determined and then cells were stimulated with a cocktail containing 10 µmol/L forskolin, 1 mmol/L isobutylmethylxanthine and 100 µmol/L cpt-cAMP (in 1% v/v DMSO). The stimulation cocktail strongly activates PKA and consequently, maximally phosphorylates/activates CFTR. Supernatant iodide levels were determined over seven consecutive 1-minute intervals post-stimulation. Consistent with previous results (18), efflux was evident within the first minute and was reliably maximal between 60-120 seconds. The maximum efflux rate, therefore, was calculated using the measured efflux level between 60-120 seconds post-stimulation (18).

### Western Blotting

Western blots for CFTR were completed as previously described (12,18). Cerebral artery lysates were prepared by grinding artery samples in lysis buffer containing 50 mM Tris (pH 7.3), 150 mM NaCl, 2 mM ETDA, 0.1% Triton-X-100, 0.1% SDS and protease inhibitors; the same lysis buffer was used to prepare cell culture lysates. Following lysis, the samples were centrifuged (10 minutes at 13,500 g; at 4oC) to remove insoluble material. Immediately prior to polyacrylamide electrophoresis, additional SDS (to 2% final concentration), glycerol (to 2% final concentration), β-mercaptoethanol (to 2% final concentration) and dithiothreitol (2 mM final concentration) were added. Proteins were separated electrophoretically on 7% acrylamide gels and transferred onto polyvinylidene difluoride (PVDF) membranes. The membranes were blocked for 30-40 minutes in 5% non-fat skim milk (in phosphate-buffered saline containing 1% Tween 20 (PBST); 137 mM NaCl, 2.7 mM KCI, 10 mM Na2HPO4, 1.76 mM K2HPO4; pH 7.4). Primary antibody treatments included: (i) Rabbit polyclonal anti-CFTR (1:1,000 in 5% milk/PBST; *Cell Signaling Technology* via *New England Biolabs Canada;* Whitby, Canada; cat# 2269); cerebral artery and vascular smooth muscle cell lysates. (ii) Mouse monoclonal anti-human CFTR (1:20,000 dilution in 5% milk/PBST; "antibody 596"); baby hamster kidney fibroblast cell lysates. (iii) Mouse monoclonal anti-α-tubulin (1:5,000 in 5% milk/PBST; clone DM1A; *Cell Signaling Technology* via *New England Biolabs Canada;* cat# 3873).

The primary antibodies were conjugated with either peroxidase-labeled donkey anti-rabbit IgG *(GE Healthcare Amersham* (Piscataway, USA) cat# NA934) or peroxidase-labeled goat anti-mouse IgG (*GE Healthcare Amersham* cat# NA931) secondary antibodies. A standard chemiluminescence procedure (Westar ETA C; VPQ Scientific, Toronto, Canada) was used to expose X-ray film or collect digital images (ChemiDoc; Bio-Rad Laboratories; Mississauga, Canada). Developed films were evaluated densitometrically using "Image J" software (freely available from the NIH); digital images were evaluated with Image Lab software (Bio-Rad).

### RNA Isolation and Reverse Transcription

Resistance artery RNA was isolated with *Norgen Biotek* (Thorold, Canada) "Total RNA Purification Micro" spin columns, using the proteinase K digestion and DNA removal procedures, as directed by the manufacturer's instructions. The eluted RNA was quantified with an *Agilent Technologies* RNA 6000 Pico Kit and Bioanalyzer; the analysis confirmed that high-quality RNA was retrieved from the artery tissue samples (RNA Integrity [RIN]: Cerebral=8.4±0.1, n=5; Cremaster=8.2±0.4, n=6). Isolation of RNA from Baby Hamster Kidney fibroblast cells followed the same procedure, with the omission of the proteinase K digestion step. In all cases, RNA was converted to cDNA using a "Superscript III" reverse transcription kit (*Invitrogen Life Technologies;* Burlington, Canada), according to the manufacturer's directions. Residual RNA was removed by incubating the resulting cDNA with RNAse H (0.125 U/µl; *New England Biolabs Canada;* Whitby, Canada).

### Quantitative PCR

Quantitative PCR was performed using an *Applied Biosystems* ViiA ^{™} 7 Real Time PCR system and Power SYBR^{®} Green PCR master mix (both distributed by *Invitrogen Life Technologies).* Each primer set was rigorously validated to ensure specificity and comparable efficiency. Gene targets were assessed in triplicate, using 1 ng of cDNA generated from the
reverse transcription; negative controls received water. The PCR amplification consisted of 10 minutes denaturation at 95oC, followed by 40 cycles of amplification (15 s at 95°C + 60 s at 60°C). Following amplification, the amplicons were melted: the resulting dissociation curve confirmed the production of single product. Transcript expression levels in mouse tissues were calculated from the DCt values relative to the standard housekeeping gene hydroxymethylbilane synthase (HMBS). To confirm that HMBS was reliable for normalization, transcript expression levels were also calculated from the DCt values relative to glucose-6-phosphate dehydrogenase (G6PD), which returned similar results. Experiments involving baby hamster kidney fibroblast cells used primers specific for human CFTR and hamster GAPDH, the latter serving as the housekeeping normalization gene.

### Brain Fixation and Slide Preparation for Immunohistochemistry and Fluoro-Jade staining

The development of delayed vasoconstriction and brain injury is more rapid in the experimental mouse SAH model (2-5 days post-SAH) (12,19-21), compared to what is observed clinically (4-12 days post-SAH ) (22,23). We selected the 2-days post-SAH induction time point for our injury marker assessments based on our previous work demonstrating significant neuronal injury and behavioral deficits at this time point (12). At 2 days post-SAH induction, animals were anesthetized with isoflurane; their brains were perfused with phosphate buffered saline (PBS) and then perfusion fixed with phosphate-buffered paraformaldehyde (4%; pH 7.4), both via the ascending aorta. Brains were immediately dissected and cut into coronal sections 1mm posterior to the bregma. These brain sections were post-fixed in 4% paraformaldehyde (pH 7.4) for 48 hours at 4oC and then incubated in 10% sucrose (3 hours), followed by 30% sucrose overnight (at 4oC) for cyroprotection. The brain sections were then embedded in OCT (Sakura Finetek USA; Torrance, USA) on isopentane and dry ice. Cryostat sections (5µm thick coronal slices) were collected on "Tissue Path Superfrost Plus Gold" slides (Fisher Scientific; Whitby, Canada) and stored at -80°C until used.

### Fluorescent Immunohistochemistry

All samples were pre-treated with 10-minute proteinase (20µg/ml; Promega; Madison, USA), followed by a standard 60-minute blocking procedure with either 10% normal donkey serum (Jackson ImmunoResearch Laboratories; West Grove, USA) in PBS or 10% goat serum (Invitrogen Life Technologies; Burlington, Canada) in PBS containing 1% bovine serum albumin. Brain slices were incubated with monoclonal rabbit anti-human active cleaved caspase-3 (clone C92-605; 1:1000 dilution; BD Biosciences Canada; Mississauga Canada) and conjugated with Alexa Fluor 488 goat anti-rabbit IgG (Invitrogen). Both antibodies were diluted in Can Get Signal^{®} immunoreaction enhancer solution and used an incubation time of 1 hour at room temperature. Specimens were washed and mounted with CC MountÔ.

### Fluoro-Jade staining

Brain slices were serially incubated with 1% NaOH / 80% ethanol (5 minutes), 70% ethanol (2 minutes), distilled water (2 minutes) and 0.06% potassium permanganate (10 minutes). After washing with deionized water, brain slices were stained with 0.0004% Fluoro-jade B (Histo-Chem Inc., Jefferson, USA) in 0.1% acetic acid (15 minutes). The samples were then washed with deionized water, dried and cleared by immersion in xylene for 1 minute. Slides were mounted using DPX mounting
medium (Sigma).

### Digital Imaging and assessment of brain damage by cell counting

Digital immunofluorescence images were acquired using a Zeiss LSM 710 confocal laser-scanning microscope in conjunction with Zeiss ZEN 2010 software (vessel images). Overlays were constructed with freely-available ImageJ 1.44p software (National Institutes of Health, USA). Two independent assessors counted caspase-3 and Fluoro-Jade positive cells under blinded conditions, as previously described (12). For each animal, counts were obtained from the cortical region of a single coronal brain slice 1mm posterior to the bregma (200x magnification), which includes the left and right temporal and parietal lobes. The number of positive cells counted by each assessor was averaged, yielding a mean positive cell count/animal that was then used for statistical analyses.

### Histological Analysis of Dendrite Morphology:

Brains were perfused with heparinized saline via transcardiac perfusion, isolated and processed/stained utilizing a commercially available Rapid GolgiStain kit (FD NeuroTechnologies Inc.; Columbia, USA). The kit uses a modified Golgi-Cox staining procedure originally described by Glaser and Van der Loos (24). Briefly, isolated brain tissue samples were immersed in a kit-provided solution containing mercuric chloride, potassium dichromate and potassium chromate in the dark for 8 days; this was followed by a 6-day incubation with tissue protection solution. The brain samples were then washed, embedded in 4% low gelling agarose, sectioned in the coronal plane (150 µm thickness; Campden Instruments 7000smz-2 vibrating microtome) and mounted onto gelatin-coated glass slides. The staining procedure was then completed using kit-provided developing/staining reagents, according to the manufacturer's instructions. Neurons and dendritic segments were imaged with stereology-based NIS Elements AR software on a Nikon Eclipse Ti2 microscope possessing motorized X-, Y-, and Zfocus for high-resolution image acquisition (Nikon Instruments Europe; Amsterdam, The Netherlands). We analyzed both the basal and apical dendrite networks of pyramidal cortical neurons from the frontal cortex, using a quantitative approach originally described by Sholl (25). We utilized Image J software
coupled with the semi-automated Simple Neurite Tracer plugin (both freely available from the NIH) to identify and digitally isolate the dendrite networks of a single cortical neuron from a 200x magnification hyperstack image. The center of the soma was marked with the pointer tool; dendrite morphology was then characterized by Sholl analysis (see https://imagej.net/Sholl_Analysis, version 3.7.4), using 5 µm intervals to a maximal radius of 300 µm. This analysis characterizes dendrite morphology in terms of dendrite intersections (i.e., branching) and dendrite length. For each treatment group, 2-4 neurons per mouse from 4 mice were analyzed under blinded conditions. To assess dendritic spine density (i.e., the number of small protrusions found on dendrites), we imaged 3rd branch order dendritic segments at 100x magnification. Spine density was measured as the number of spines per segment for the following segments: 20-50 µm from soma; 30-60 µm from soma; 50-80 µm from soma; and 60-100 µm from soma). This "sliding measurement" approach permits an assessment of how spine density changes over the length of dendritic branch. For each treatment group, spine density was measured in 2-4 pyramidal cortical neurons (2-4 third order branches per neuron) per mouse from 3-4 mice per group, under blinded conditions.

As described above, the CFTR corrector C18 and the anti-human CFTR antibody (designated "596") were acquired through the *Cystic Fibrosis Foundation Therapeutics* Chemical and Antibody Distribution Programs. All other reagents used are commercially available as outlined above.

**Animals:** The Institutional Animal Care and Use Committees at the University of Toronto and the University Health Network (UHN) approved all animal care and experimental protocols. Commercially available male wild-type mice (2-3 months; C57BL/6N) were purchased from Charles River Laboratories (Montreal, Canada). Male mice homozygous for the ΔF508 CFTR mutation (CFTR^{tm1EUR}; designated "ΔF508" in the present study) (11), CFTR gene deletion (CFTR^{tm1Unc}; designated CFTR^{-/-}) and the complimentary wild-type control littermates were obtained from an established colony at the *Hospital for Sick Children,* Toronto (all CFTR⁻¹⁻, CFTR^{.ΔF508} and littermates are mixed strains). All mice were housed under a standard 14h:10h light-dark cycle, fed normal chow and had access to water *ad libitum.*

**Myocardial infarction:** HF was induced by surgical ligation of the left anterior descending coronary artery (3). Briefly, mice were anaesthetized with isoflurane, intubated with a 20-gauge angiocatheter and ventilated with room air. Under sterile conditions, the thorax and pericardium were opened, and the left anterior descending coronary artery was permanently ligated with 7-0 silk suture (Deknatel; Fall River, USA). In sham-operated controls, the thorax and pericardium were opened, but the left anterior descending coronary artery was not ligated. Following the procedure, the chest was closed and the mice were extubated upon spontaneous respiratio
n. Posterior cerebral arteries (PCAs) were isolated 4-6 weeks post-infarction.

**Induction of Subarachnoid Hemorrhage:** A well-characterized model of experimental SAH (6) was used. Briefly, each mouse was anaesthetized (isoflurane) and its head was fixed in a stereotactic frame; a 7mm incision was made along the midline of the anterior scalp and 0.9mm hole drilled into the skull 4.5mm anterior to the bregma. A spinal needle was advanced to the chiasmatic cistern: 80µl of arterial blood was injected into the intracranial space over 10 seconds. The injected blood was obtained from a separate wild-type donor mouse (via cardiac puncture) immediately prior to injection and did not contain anticoagulants. Following injection, the scalp incision was closed. Buprenorphine (0.05mg/kg; 0.5-1.0ml volume) was administered twice/day (initiated immediately following the SAH surgical procedure). Sham-operated animals underwent an identical surgical procedure, with sterile saline injected instead of blood. Olfactory cerebral arteries were isolated at 2 days post-SAH induction.

**Isolation and functional assessment of resistance arteries:** Mouse olfactory (a first branch of the anterior cerebral artery) and PCAs were carefully dissected, cannulated onto micropipettes, stretched to their *in vivo* lengths and pressurized to 45 mmHg, as previously described (3,6); mouse skeletal muscle resistance arteries were dissected from the cremaster muscle, cannulated and pressurized to 60 mmHg. All functional experiments were conducted in 3-morpholinopropanesulfonic acid (MOPS) buffered saline at 37°C with no perfusion. Vasomotor responses to phenylephrine (5 µmol/L for posterior cerebral arteries, 10 µmol/L for cremaster skeletal muscle arteries) provided an assessment of vessel viability at the beginning and end of each experiment. Arteries failing to show ≥25% constriction to phenylephrine were excluded.

Myogenic responses were elicited by step-wise 20mmHg increases in transmural pressure from 20 mmHg to 80mmHg (olfactory arteries) or 100 mmHg (posterior cerebral arteries and cremaster skeletal muscle resistance arteries). At each pressure step, vessel diameter (dia_{active}) was measured once a steady state was reached (5 min). Vessels requiring treatment (e.g., CFTR₍ᵢₙₕ₎-172) were incubated with the reagent in MOPS for 30 min and myogenic responses were then assessed in the presence of that reagent. Following completion of all dia_{active} measurements, MOPS buffer was replaced with a Ca²⁺-free version and maximal passive diameter (diaₘₐₓ) was recorded at each pressure step.

Myogenic tone was calculated as the percent constriction in relation to the maximal diameter at each respective transmural pressure: tone (% of diaₘₐₓ) = [(diaₘₐₓ-dia_{active})/diaₘₐₓ]x100, where dia_{active} is the vessel diameter in MOPS containing Ca²⁺ and diaₘₐₓ is the diameter in Ca²⁺-free MOPS. Analyses of vasomotor responses to phenylephrine used the same calculation, only in this case, dia_{active} represents the vessel diameter at steady state following application of the given agent and diaₘₐₓ represents the maximal diameter measured under Ca²⁺-free conditions.

**Magnetic resonance imaging-based cerebral blood flow measurements: A** a non-invasive magnetic resonance imaging (MRI)-approach (FAIR technique) was uitilized to evaluate cerebral perfusion, as previously described (6). Briefly, the FAIR technique isolates perfusion as an accelerated T₁ signal relaxation. MRI signals (*Bruker Corporation* Biospec 70/30 USR; Ettlingen, Germany) were acquired from vertical sections of the fore, middle, and hind-brain, which correspond to the anterior, mixed, and posterior circulations. FAIR images were evaluated for designated regions of interest (region placement is displayed in Figure 8) using standardized algorithms and image processing procedures (MIPAV; National Institutes of Health, Bethesda, USA; http://mipav.cit.nih.gov/).

**Magnetic resonance imaging-based edema measurement:** Edema was assessed by quantitative T₂ mapping (12), using a 7 Tesla micro-MRI system (*Bruker Corporation* Biospec 70/30 USR; Ettlingen, Germany). The T₂ mapping acquisition generated quantitative T₂ maps in 9 contiguous 2D axial slices, with 1 mm thickness, covering the volume from the fore-brain through to the hind-brain. T₂ maps were generated from T₂-weighted images at echo times from 12 to 384 ms, using in-line Bruker software, via linear regression of the logarithmically-transformed signal magnitudes and the echo times on a per voxel basis. The T₂ values were extracted using MIPAV software, using manually drawn regions-of-interest placed within the fore-, middle- and hind-brain T₂ maps.

**Neurological Function Assessment:** Neurological function was assessed utilizing the modified Garcia score, as previously described (6). The neurological assessment consists of 6 domains: spontaneous activity, spontaneous movement of all 4 limbs, forepaw outstretching, climbing, body proprioception and response to vibrissae touch. Two blinded observers conducted the neurological assessment 2 days after SAH. The maximum score is 18, indicative of normal neurological function.

**Statistics:** All data are expressed as means ± SEM, where n is the number of independent measures (i.e., samples, vessels assessments or experimental subjects). For statistical comparisons, a non-parametric Mann-Whitney test with exact p-value computation was utilized for the comparison of two independent groups. For comparison of multiple independent groups, a non-parametric one-way ANOVA (Kruskal-Wallis) was utilized, followed by a Dunn's post-hoc test. For the assessment of myogenic responses and dose-response relationships, data were analyzed with a two-way ANOVA, followed by a Tukey's post-hoc test. Differences were considered significant at P<0.05. All data presented in Figure 6 were collected under blinded conditions; all other data presented in this study did not require blinding and were not collected under blinded conditions.

The following references are referred to in the above METHODS AND REAGENTS section:
1. Cui L, Aleksandrov L, Chang XB, et al. Domain interdependence in the biosynthetic assembly of CFTR. J Mol Biol 2007;365:981-94.
2. van Doorninck JH, French PJ, Verbeek E, et al. A mouse model for the cystic fibrosis delta F508 mutation. EMBO J 1995;14:4403-11.
3. Van Goor F, Straley KS, Cao D, et al. Rescue of DeltaF508-CFTR trafficking and gating in human cystic fibrosis airway primary cultures by small molecules. Am J Physiol Lung Cell Mol Physiol 2006;290:L1117-30.
4. French PJ, van Doorninck JH, Peters RH, et al. A delta F508 mutation in mouse cystic fibrosis transmembrane conductance regulator results in a temperature-sensitive processing defect in vivo. J Clin Invest 1996;98:1304-12.
5. Zeiher BG, Eichwald E, Zabner J, et al. A mouse model for the delta F508 allele of cystic fibrosis. J Clin Invest 1995;96:2051-64.
6. Wilke M, Buijs-Offerman RM, Aarbiou J, et al. Mouse models of cystic fibrosis: phenotypic analysis and research applications. J Cyst Fibros 2011;10 Suppl 2:S152-71.
7. Lavelle GM, White MM, Browne N, McElvaney NG, Reeves EP. Animal Models of Cystic Fibrosis Pathology: Phenotypic Parallels and Divergences. Biomed Res Int 2016;2016:5258727.
8. Nilius B, Droogmans G. Amazing chloride channels: an overview. Acta Physiol Scand 2003;177:119-47.
9. Hoefer J, Azam MA, Kroetsch JT, et al. Sphingosine-1-phosphate-dependent activation of p38 MAPK maintains elevated peripheral resistance in heart failure through increased myogenic vasoconstriction. Circ Res 2010;107:923-33.
10. Meissner A, Yang J, Kroetsch JT, et al. Tumor Necrosis Factor-α-Mediated Downregulation of the Cystic Fibrosis Transmembrane Conductance Regulator Drives Pathological Sphingosine-1- hosphate Signaling in a Mouse Model of Heart Failure. Circulation 2012;125:2739 50.
11. Yang J, Hossein Noyan-Ashraf M, Meissner A, et al. Proximal Cerebral Arteries Develop Myogenic Responsiveness in Heart Failure via Tumor Necrosis Factor-α-Dependent Activation of Sphingosine-1-Phosphate Signaling. Circulation 2012;126:196-206.
12. Yagi K, Lidington D, Wan H, et al. Therapeutically Targeting Tumor Necrosis Factor-α/Sphingosine-1-Phosphate Signaling Corrects Myogenic Reactivity in Subarachnoid Hemorrhage. Stroke 2015;46:2260-70.
13. Kim SG. Quantification of relative cerebral blood flow change by flow-sensitive alternating inversion recovery (FAIR) technique: application to functional mapping. Magn Reson Med 1995;34:293-301.
14. Herscovitch P, Raichle ME. What is the correct value for the brain--blood partition coefficient for water? J Cereb Blood Flow Metab 1985;5:65-9.
15. Foley LM, Hitchens TK, Kochanek PM, Melick JA, Jackson EK, Ho C. Murine orthostatic response during prolonged vertical studies: effect on cerebral blood flow measured by arterial spinlabeled MRI. Magn Reson Med 2005;54:798-806.
16. Foltz WD, Porter DA, Simeonov A, et al. Readout-segmented echo-planar diffusion-weighted imaging improves geometric performance for image-guided radiation therapy of pelvic tumors. Radiother Oncol 2015;117:525-31.
17. Sharma M, Benharouga M, Hu W, Lukacs GL. Conformational and temperature-sensitive stability defects of the delta F508 cystic fibrosis transmembrane conductance regulator in post-endoplasmic reticulum compartments. J Biol Chem 2001;276:8942-50.
18. Malik FA, Meissner A, Semenkov I, et al. Sphingosine-1-Phosphate Is a Novel Regulator of Cystic Fibrosis Transmembrane Conductance Regulator (CFTR) Activity. PLoS One 2015;10:e0130313.
19. Lin CL, Calisaneller T, Ukita N, Dumont AS, Kassell NF, Lee KS. A murine model of subarachnoid hemorrhage-induced cerebral vasospasm. J Neurosci Methods 2003;123:89-97.
20. Parra A, McGirt MJ, Sheng H, Laskowitz DT, Pearlstein RD, Warner DS. Mouse model of subarachnoid hemorrhage associated cerebral vasospasm: methodological analysis. Neurol Res 2002;24:510-6.
21. Aum DJ, Vellimana AK, Singh I, et al. A novel fluorescent imaging technique for assessment of cerebral vasospasm after experimental subarachnoid hemorrhage. Sci Rep 2017;7:9126.
22. Brilstra EH, Rinkel GJ, Algra A, van Gijn J. Rebleeding, secondary ischemia, and timing of operation in patients with subarachnoid hemorrhage. Neurology 2000;55:1656-60.
23. Macdonald RL. Delayed neurological deterioration after subarachnoid haemorrhage. Nat Rev Neurol 2014;10:44-58.
24. Glaser EM, Van der Loos H. Analysis of thick brain sections by obverse-reverse computer microscopy: application of a new, high clarity Golgi-Nissl stain. J Neurosci Methods 1981;4:117-25.
25. Sholl DA. Dendritic organization in the neurons of the visual and motor cortices of the cat. J Anat 1953;87:387-406.

### RESULTS

### Deficient CFTR function drives reduced cerebral perfusion

Previous work demonstrated that CFTR prominently regulates cerebral artery myogenic tone and identified a strong *correlation* between cerebral artery CFTR protein expression and cerebral perfusion (4,6). The mechanistic concept, therefore, proposes that correcting deficient cerebrovascular CFTR activity (i.e., reduced S1P transport due to decreased CFTR expression at the cell surface) is a viable therapeutic strategy for normalizing compromised cerebral myogenic responsiveness and hence, cerebral blood flow (4).

To establish the *causative* link between cerebral artery CFTR activity and cerebral perfusion, CFTR mutant mice were utilized. Since CFTR^{-/-} mice were extremely sensitive to experimental stressors, which did not permit accurate and reproducible echocardiography / CBF measurements. Therefore, more stable CFTR^{ΔF508} mice, which have minimal cell surface CFTR expression and activity (11), were used. This underpinned the inventor's strategic decision to use CFTR^{ΔF508} mice for relating cerebral artery myogenic tone to hemodynamic parameters in a loss of function phenotype.

Vasomotor reactivity in posterior cerebral arteries (PCAs) was assessed, in order to directly compare the CFTR^{ΔF508} phenotype to the previously published characterization of PCA responses from CFTR knockout mice (4). As expected, PCAs from CFTR^{ΔF508} mice display augmented myogenic tone, relative to wild-type littermates (Figure 8A); the magnitude of the augmentation is qualitatively similar to that found for CFTR^{-/-} mice (4). Phenylephrine responses display an upward shift due to the enhanced myogenic tone (Figure 8B); however, the EC₅₀ values are not different (log EC₅₀ WT: -5.83±0.21, n=5 from 3 mice;
log EC₅₀ CFTR^{ΔF508}: -6.00±0.04, n=6 from 4 mice; Mann-Whitney test P=N.S.) and the difference between the curves is eliminated by correcting for the difference in basal tone (i.e., myogenic tone at 45 mmHg; Figure 8C). The ΔF508 mutation, therefore, augments myogenic responsiveness, but does not alter general contractility. CBF is significantly lower in CFTR^{ΔF508} mice (Figure 8D); however, neither CO (Figure 8E) nor TPR (Figure 8F) are affected by the mutation. Based on these observations, the cerebral perfusion deficit is clearly vascular in origin.

The CFTR^{ΔF508} mutation does not change TPR, indicating that not all vascular beds are subject to CFTR-dependent regulation. Since TPR is primarily generated and regulated by skeletal muscle resistance arteries (14), it was assessed whether CFTR influences myogenic tone in mouse cremaster skeletal muscle resistance arteries. CFTR mRNA expression is approximately 10-fold lower in wild-type mouse cremaster skeletal muscle resistance arteries, relative to cerebral arteries (Figure 1G). Inhibiting CFTR activity *in vitro* (100 nmol/L CFTR₍ᵢₙₕ₎-172, 30 minutes) does not affect wild-type cremaster artery myogenic tone (Figure 1H), which contrasts recent observations that CFTR₍ᵢₙₕ₎-172 augments olfactory cerebral artery myogenic tone (6). Unlike PCAs (4), CFTR gene deletion does not augment myogenic tone in cremaster skeletal muscle resistance arteries (Figure 11); in fact, myogenic tone is mildly attenuated and corresponds to lower MAP (Figure 9). Phenylephrine dose-response relationships are not altered by either CFTR inhibition *in vitro* or CFTR gene deletion (Figure 10).

In summary, the present CFTR^{ΔF508} experiments establish: (i) a *causative* link between CFTR expression, PCA myogenic responsiveness and CBF; (ii) that CFTR modulates myogenic tone in specific vascular beds; and (iii) that CFTR does not modulate skeletal muscle resistance artery tone, which is notable, because these arteries prominently contribute to TPR. Since HF and SAH down-regulate cerebral artery CFTR expression concomitant with microvascular dysfunction (4,6), these data provide a strong mechanistic foundation for using CFTR-modulating medications to specifically correct cerebrovascular dysfunction and CBF deficits in this pathological setting.

### C18 increases CFTR activity following TNF-dependent down-regulation

Current CFTR-modulating medications, identified via high throughput drug screens, are experimentally and clinically utilized to mitigate the effects of cystic fibrosis. In According to the present example C18 was selected for the studies, since it was demonstrated earlier that it directly interacts with and stabilizes wild-type CFTR, thereby increasing CFTR expression at the plasma membrane (15).

According to the present invention, it was firstly confirmed that C18 is capable of increasing both mouse and human CFTR expression. Cerebral arteries isolated from naive mice injected with C18 (3 mg/kg daily for 2 days) display higher CFTR protein expression levels, relative to vehicle-treated controls (Figure 2A); CFTR mRNA expression is not affected by C18 (Figure 2B). To confirm C18's effect on *human* CFTR, a heterologous expression system of baby hamster kidney fibroblast cells was used, stably expressing a human CFTR construct. C18 treatment (6 µmol/L; 24 h) more than doubles CFTR protein expression in baby hamster kidney fibroblast cells (Figure 2C); C18 has no affect on CFTR mRNA expression in this system (Figure 2D). Collectively, the data are consistent with previous observations that C18 exerts direct stabilizing effects on CFTR protein expression (15) and demonstrate its efficacy against both murine and human CFTR.

Next it was assessed whether C18 treatment increases CFTR abundance in a cell culture setting that reasonably mimics the relevant pathophysiological mechanisms and cellular environment. As was previously demonstrated (4), TNF (10 ng/ml) reduces CFTR protein expression in primary vascular smooth muscle cells and thus, mimics the TNF-dependent CFTR down-regulation observed in HF (4) and SAH (Figure 2E) (6). As expected, the decline in CFTR expression correlates with attenuated FITC-S1P uptake (i.e., a CFTR-dependent process (4); Figure 2F) and forskolin-stimulated iodide efflux (i.e., a classic measure of CFTR activity (13); Figure 2G). C18 treatment (6 µmol/L; 24 h co-incubation with 10 ng/ml TNF following 24 h incubation with 10 ng/ml TNF) reverses the decline in CFTR expression (Figure 2E) and fully restores FITC-S1P uptake (Figure 2F) and iodide efflux (Figure 2G). These results perfectly align with the prediction of the present invention that under conditions where CFTR is down-regulated, C18's proteostatic effect increases total CFTR expression and restores its activity.

### C18 treatment rectifies deficient cerebral blood flow in heart failure

As previously documented by Meissner *et al.* (4), the induction of HF stimulates a significant reduction in cerebral artery CFTR protein expression (Figure 3A) that coincides with a marked augmentation in PCA myogenic tone (Figure 3B). As predicted by the data in Figure 2, C18 treatment *in vivo* (3 mg/kg daily for 2 days) restores CFTR expression in cerebral arteries isolated from mice with HF (Figure 3A) and concomitantly normalizes PCA myogenic tone (Figure 3B). The attenuation of myogenic tone associates with an expected shift in phenylephrine responsiveness, due to the reduction in basal tone; however, the EC₅₀ values are not different (log EC₅₀ Sham: -6.30±0.14, n=6 from 4 mice;
log EC₅₀ HF: -6.25±0.07, n=5 from 3 mice; log EC₅₀ HF+C18: -5.94±0.21, n=5 from 4 mice; Kruskal-Wallis test P=N.S.) and the curves overlap after correction for the difference in in basal tone (Figure 11). C18 treatment does not affect PCA myogenic tone or phenylephrine responses in sham-operated mice (Figure 12). Using PCAs isolated from CFTR knockout mice, the present invention confirmed that C18 mediates its attenuating effect on myogenic tone by targeting CFTR. As expected, PCAs from CFTR knockout mice display augmented myogenic tone that is not susceptible to *in vivo* C18 treatment (Figure 3C); phenylephrine responses are not affected by C18 treatment (Figure 13).

At the systemic level, the induction of HF significantly reduces CO relative to sham-operated controls (Figure 3D; measured at 6 weeks post-infarction); TPR increases as a compensatory response (Figure 3E) to prevent a large reduction in MAP (Figure 3F) (3,4). Despite the relatively small reduction in MAP, CBF is markedly reduced (Figure 3G). C18 treatment *in vivo* restores CBF in mice with HF (Figure 3G), faithfully correlating to the normalization of PCA myogenic tone (Figure 3B). C18 does not ameliorate the cardiac injury induced by the left anterior descending coronary artery ligation procedure : the improvement in CBF, therefore, can be unambiguously attributed to a vascular mechanism. Further, since C18 has no effect on TPR or MAP (Figure 3), the C18-mediated restoration of CBF must be *a localized* microvascular effect that is independent of changes to systemic hemodynamic parameters. This therapeutic profile perfectly aligns with previously described etanercept intervention (3).

In addition to maintaining constant perfusion when systemic pressure fluctuates, the myogenic response also protects fragile capillary beds from damaging pressure levels (16) and maintains capillary hydrostatic pressure at levels that minimize edema formation (17). In this context, therapeutically reducing cerebral artery myogenic tone, even from the augmented level that occurs in HF, could potentially cause the counter-productive side effect of cerebral edema formation. Therefore, non-invasive imaging for edema was used to confirm that neither HF nor C18 treatment in the context of HF induces evident edema in any region of the brain (Figure 4).

### C18 treatment rectifies deficient cerebral blood flow in SAH

Previous work demonstrates that SAH has a similar cerebrovascular phenotype as HF, in that (i) cerebral artery CFTR protein expression is reduced; (ii) cerebral artery myogenic tone is augmented; and (iii) cerebral perfusion is reduced (6). As in HF (Figure 3), C18 treatment *in vivo* (3 mg/kg daily for 2 days) restores CFTR expression in cerebral arteries from mice with SAH (Figure 5A) and concomitantly normalizes olfactory cerebral artery myogenic tone (Figure 5B). The attenuation of myogenic tone associates with an expected baseline shift in phenylephrine responsiveness, due to the reduction in basal tone; however, the EC₅₀ values are not different (log EC₅₀ Sham: -5.52±0.23, n=5 from 3 mice; log EC₅₀ SAH: -6.01±0.16, n=6 from 6 mice; log EC₅₀ SAH+C18: -6.01±0.31, n=5 from 4 mice; Kruskal-Wallis test P=N.S.) and the curves overlap after correction for the difference in in basal tone (Figure 14). C18 treatment does not affect olfactory cerebral myogenic tone or phenylephrine responses in sham-operated mice (Figure 15). Using olfactory cerebral arteries isolated from CFTR knockout mice, the present invention confirms that C18 mediates its attenuating effect on myogenic tone by targeting CFTR: as expected, olfactory cerebral arteries from CFTR knockout mice display augmented myogenic tone that is not susceptible to *in vivo* C18 treatment (Figure 5C); phenylephrine responsiveness in these arteries is not affected by C18 treatment (Figure 16). Importantly, *in vivo* C18 treatment restores CBF (Figure 5D), once again correlating with the normalization of olfactory cerebral artery myogenic tone (Figure 5B).

### Iumacaftor treatment rectifies deficient cerebral blood flow in SAH

The C18 data are complemented with interventions utilizing the CFTR corrector lumacaftor, a clinically relevant C18 analogue that is FDA approved for treating cystic fibrosis, in combination with the CFTR potentiator Ivacaftor (i.e., Orkambi^{®}). It was first confirmed that lumacaftor is capable of increasing both mouse and human CFTR expression: as observed for C18 (Figure 2), lumacaftor increases CFTR protein expression in mouse cerebral arteries (mice injected with 3 mg/kg daily for 2 days) and baby hamster kidney fibroblast cells stably expressing human CFTR (Figure 6). In both settings, CFTR mRNA expression was unaffected, indicating a non-transcriptional mechanism (Figure 6). Consistent with the present C18 data in SAH (Figure 5), lumacaftor treatment *in vivo* (3 mg/kg daily for 2 days) restores CFTR expression in cerebral arteries from SAH mice (Figure 6) and concomitantly normalizes olfactory cerebral artery myogenic tone (Figure 6) and cerebral perfusion (Figure 6). lumacaftor does not affect phenylephrine responsiveness (Figure 17) and consequently, the EC₅₀ values are not different (log EC₅₀ Sham: -5.79±0.09, n=7 from 4 mice;
log EC₅₀ SAH: -5.93±0.19, n=6 from 3 mice; log EC₅₀ SAH+Lum: -5.90±0.18, n=7 from 4 mice; Kruskal-Wallis test P=N.S.).

### C18 and lumacaftor reduce neuronal injury in SAH

The neuronal injury induced by SAH can be easily characterized with standard histological techniques (Fluoro-Jade and activated caspase-3 staining) and simple neurological testing (Modified Garcia Score) (6): these methods were utilized to determine whether the restoration of normal myogenic responsiveness and CBF correlate with improved neurological outcome. Indeed, both C18 and lumacaftor dramatically attenuate neuronal injury in SAH, as assessed by activated caspase-3 and Fluoro-Jade staining (Figure 7); the neuronal injury reduction in C18-treated mice correlates with improved neurological function (Figure 7E). Specifically, SAH mice scored lower than sham-operated mice on the modified Garcia neurological function test; C18-treated SAH mice, however, had neurological function scores comparable to sham-operated mice.

### DISCUSSION

Previous work advanced the initial discovery that CFTR inhibition augments microvascular myogenic tone into a novel mechanistic concept (4,6): at its core, CFTR critically regulates S1P degradation and thus, prominently regulates cerebrovascular tone in health and disease (4). The present investigation applies this knowledge to treat HF and SAH. By the present invention, a new class of therapeutics to cardiovascular medicines is introduced. The present invention presents *in vivo* verification that CFTR is a cerebrovascular regulator and confirms that it can be exploited to improve cerebral perfusion in both HF and SAH. As the first new microvascular drug-target to emerge in years, therapeutics that enhance CFTR function may represent an untapped resource for managing a broad array of pathologies that induce cerebrovascular dysfunction and cerebral perfusion deficits.

Proximal PCAs were strategically selected for the HF studies presented herein and olfactory cerebral arteries for the SAH studies according to the present Example, in order to directly compare the CFTR-targeted interventions to previous work (3,4,6). Although PCAs and olfactory arteries originate from distinct regions of the cerebral microcirculation (i.e., posterior and anterior, respectively) and display differences in their baseline myogenic tone curves, they nevertheless behave similarly in terms of the pathological signaling mechanisms that augment myogenic responsiveness (3,4,6). The comparable success of the CFTR-targeted interventions in PCAs and olfactory arteries suggests that CFTR regulates vascular reactivity broadly throughout the cerebral microcirculation.

Clearly, CFTR does not regulate vascular reactivity in all vascular beds, as revealed by the comparisons of (i) cerebral and skeletal muscle resistance arteries, (ii) TPR measurements CFTR mutant and wild-type mice and (iii) the lack of effect of C18 treatment on TPR. Differences in CFTR expression, as measured by quantitative PCR, ostensibly explain why CFTR inhibition regulates myogenic tone in cerebral arteries, yet plays no obvious role in skeletal muscle resistance arteries. Collectively, the present data demonstrate that CFTR therapeutics will have restricted effects that are specific to discrete microvascular beds (e.g., the cerebral microcirculation). This attribute is a prerequisite for effective blood flow redistribution and confers a significant therapeutic advantage over general vasodilators that can dangerously lower blood pressure through indiscriminant vasodilation.

Interestingly, while acute/chemical CFTR inhibition had no effect in cremaster resistance arteries, germline CFTR gene deletion slightly reduced myogenic tone in this specific microvascular bed. Since global CFTR deletion induces a complex cardiovascular phenotype (18), this mild discrepancy is attributable to an indirect and/or adaptive response. The inventors have previously demonstrated that the CFTR inhibitor's efficacy in both cell (4) and isolated artery (6) systems. Therefore, it can be concluded that CFTR plays a minimal role in modulating cremaster artery myogenic tone.

The myogenic response is the basis of CBF autoregulation, the continuous matching of vascular resistance to the prevalent transmural pressure. Thus, interventions that aim to increase pathologically reduced CBF must strive to restore normal myogenic reactivity, rather than indiscriminately dampening vasoconstriction. By specifically normalizing myogenic reactivity in HF and SAH, the present CFTR-targeted interventions are expected to preserve both CBF autoregulation and responsiveness to local vasoactive stimuli (e.g., neuro-vascular coupling) *in vivo.*

In addition to dictating cerebral perfusion through its autoregulatory function, the myogenic response also maintains capillary pressures at levels that minimize damage and edema formation (16,17). Reducing myogenic reactivity, therefore, has the potential to induce edema formation by permitting additional pressure to enter the capillary beds. On a related aspect, since S1P prominently regulates blood brain barrier permeability (19), CFTR-dependent changes in S1P signaling could cause edema through alterations in barrier function. Thus, it was crucial that to exclude edema as a serious negative effect of the present intervention. Indeed, CFTR corrector treatment (C18) does not induce edema in mice with HF. This confirms that: (i) hydrostatic pressure remains within tolerable limits (this would be expected, since the CBF and MAP measures are comparable to sham animals); and (ii) blood-brain barrier function is preserved during treatment.

Hitherto, cerebrovascular CFTR expression has never been assessed in human cerebral arteries, nor has cerebral perfusion been determined in cystic fibrosis patients. Clinically, cystic fibrosis is not associated with cognitive impairment or ischemic injury; however, this does not preclude reduced cerebral perfusion: as has been demonstrated before (3, 20), the brain can tolerate a moderate CBF reduction without crossing an obvious injury threshold. Thus, cognitive impairment should not necessarily be expected in young and otherwise relatively healthy cystic fibrosis patients. Although there is little doubt that hypoperfusion significantly contributes to cognitive decline (1) and cerebral injury (21), other "hits to the system", including aging and inflammatory responses elicited by heart disease or SAH, are required to push clinically silent CBF reductions across the injury threshold.

Access to viable human cerebral artery specimens is inherently difficult: thus, the present inventors relied on their translational work involving systematic human/mouse comparisons of mesenteric and skeletal muscle artery CFTR expression (22) to ascertain whether CFTR is likely to be expressed in human cerebral arteries. In human mesenteric arteries, CFTR protein is present and its functional inhibition leads to an expected augmentation of myogenic responsiveness (22): it is therefore confirmed that CFTR is a modulator of human resistance artery myogenic reactivity. In contrast to human mesenteric arteries, human skeletal muscle resistance arteries have no detectable CFTR protein and are consequently, insensitive to CFTR inhibition (22). Since the functional profile for mesenteric and skeletal muscle arteries for humans and mice overlaps (i.e., CFTR modulates mesenteric, but not skeletal muscle resistance arteries; Figure 1 (4,22)), it can be extrapolated that the functional profile also overlaps in the cerebral microcirculation. In the absence of direct assessments, this is, at present, the most reasonable basis to predict the effect of CFTR modulators in the human cerebrovascular setting.

Corrector compounds are currently used to treat cystic fibrosis stemming from the ΔF508 CFTR mutation: these compounds assist in chaperoning misfolded mutant CFTR proteins to the plasma membrane. Neither the pathology of deficient CFTR trafficking nor the therapeutic intervention of chaperoning applies to HF and SAH, where a transcription-based reduction in CFTR expression occurs. Thus, it was crucial to confirm that CFTR modulators, preferably CFTR correctors, in particular C18 and lumacaftor, increase *wild-type* CFTR abundance in an experimental setting where CFTR protein expression is down-regulated. Indeed, the present data show that CFTR modulators, preferably CFTR correctors, in particular C18 and lumacaftor, also increase wild-type CFTR abundance, by a non-transcriptional mechanism. This proteostatic effect stabilizes cell surface-localized CFTR against internalization and subsequent degradation (15). Over time, stabilizing cell surface CFTR elevates overall CFTR expression levels, since less cell surface CFTR is internalized and routed to degradation mechanisms. The therapeutic restoration of CFTR expression in the disease models utilized in the present invention coincides with an attenuation of myogenic tone, the normalization of CBF and in SAH, a reduction in neuronal injury..

Since CFTR is a critical regulatory protein in several organs, most notably the lung, the impact of CFTR therapeutics on non-vascular tissues must also be considered. Notably, HF down-regulates CFTR expression in lung terminal bronchiolar epithelial cells (4) and thus, CFTR decline is not restricted to the microcirculation in this setting (i.e., HF evokes "a cystic fibrosis phenotype" that potentially drives multi-organ failure). CFTR therapeutics could, therefore, possess broad and substantive benefits for HF patients, beyond the improvement of cerebral perfusion. In this context, the fact that several serious co-morbidities are common to both HF and cystic fibrosis (e.g., secondary pulmonary hypertension (24)) fuels the provocative, yet speculative contention that certain secondary pathologies in HF are either caused or aggravated by insufficient CFTR activity and therefore, correctable with CFTR therapeutics.

### CONCLUSIONS

The present invention provides the first evidence that CFTR regulates cerebrovascular reactivity and hence, cerebral perfusion: this positions CFTR as a "master switch" in the control of cerebral perfusion. HF and SAH both chronically reduce cerebral perfusion by down-regulating cerebral artery CFTR protein expression and thereby compromising autoregulatory control. The present Example demonstrates that clinically available CFTR therapeutics can restore cerebral artery CFTR expression, vascular reactivity and cerebral perfusion. Remarkably, this therapeutic effect is localized to the cerebral microcirculation, since CFTR does modulate the reactivity of peripheral arteries involved in the control blood pressure. CFTR therapeutics, therefore, emerge as valuable clinical tools to manage cerebrovascular dysfunction, impaired cerebral perfusion and neuronal injury. The present example shows the efficacy of CFTR therapeutics for the prevention and improvement of cerebral perfusion deficits, in particular caused by HF and SAH.

The following references are referred to in the present disclosure with exception to the above METHODS AND REAGENTS section of the Example to which a separate reference list applies as described in that section.
1. Román GC. Brain hypoperfusion: a critical factor in vascular dementia. Neurol Res 2004;26:454-8.
2. Román GC. Stroke, cognitive decline and vascular dementia: the silent epidemic of the 21st century. Neuroepidemiology 2003;22:161-4.
3. Yang J, Hossein Noyan-Ashraf M, Meissner A, et al. Proximal Cerebral Arteries Develop Myogenic Responsiveness in Heart Failure via Tumor Necrosis Factor-α-Dependent Activation of Sphingosine-1-Phosphate Signaling. Circulation 2012;126:196-206.
4. Meissner A, Yang J, Kroetsch JT, et al. Tumor Necrosis Factor-α-Mediated Downregulation of the Cystic Fibrosis Transmembrane Conductance Regulator Drives Pathological Sphingosine-1-Phosphate Signaling in a Mouse Model of Heart Failure. Circulation 2012;125:2739-50.
5. Meissner A, Visanji NP, Momen MA, et al. Tumor Necrosis Factor-α Underlies Loss of Cortical Dendritic Spine Density in a Mouse Model of Congestive Heart Failure. J Am Heart Assoc 2015;4:e001920.
6. Yagi K, Lidington D, Wan H, et al. Therapeutically Targeting Tumor Necrosis Factor-α/Sphingosine-1-Phosphate Signaling Corrects Myogenic Reactivity in Subarachnoid Hemorrhage. Stroke 2015;46:2260-70.
7. Murdaca G, Spanò F, Contatore M, et al. Infection risk associated with anti-TNF-α agents: a review. Expert Opin Drug Saf 2015;14:571-82.
8. Kroetsch JT, Levy AS, Zhang H, et al. Constitutive smooth muscle tumour necrosis factor regulates microvascular myogenic responsiveness and systemic blood pressure. Nat Commun 2017;8:14805.
9. Blankenbach KV, Schwalm S, Pfeilschifter J, Meyer Zu Heringdorf D. Sphingosine-1-Phosphate Receptor-2 Antagonists: Therapeutic Potential and Potential Risks. Front Pharmacol 2016;7:167.
10. Boyle MP, Bell SC, Konstan MW, et al. A CFTR corrector (lumacaftor) and a CFTR potentiator (ivacaftor) for treatment of patients with cystic fibrosis who have a phe508del CFTR mutation: a phase 2 randomised controlled trial. Lancet Respir Med 2014;2:527-38.
11. van Doorninck JH, French PJ, Verbeek E, et al. A mouse model for the cystic fibrosis delta F508 mutation. EMBO J 1995;14:4403-11.
12. Loubinoux I, Volk A, Borredon J, et al. Spreading of vasogenic edema and cytotoxic edema assessed by quantitative diffusion and T2 magnetic resonance imaging. Stroke 1997;28:419-26.
13. Malik FA, Meissner A, Semenkov I, et al. Sphingosine-1-Phosphate Is a Novel Regulator of Cystic Fibrosis Transmembrane Conductance Regulator (CFTR) Activity. PLoS One 2015;10:e0130313.
14. Henrion D. Pressure and flow-dependent tone in resistance arteries. Role of myogenic tone. Arch Mal Coeur Vaiss 2005;98:913-21.
15. Okiyoneda T, Veit G, Dekkers JF, et al. Mechanism-based corrector combination restores ΔF508-CFTR folding and function. Nat Chem Biol 2013;9:444-54.
16. Bidani AK, Griffin KA, Williamson G, Wang X, Loutzenhiser R. Protective importance of the myogenic response in the renal circulation. Hypertension 2009;54:393-8.
17. Moien-Afshari F, Skarsgard PL, McManus BM, Laher I. Cardiac transplantation and resistance artery myogenic tone. Can J Physiol Pharmacol 2004;82:840-8.
18. Van Iterson EH, Karpen SR, Baker SE, Wheatley CM, Morgan WJ, Snyder EM. Impaired cardiac and peripheral hemodynamic responses to inhaled β2-agonist in cystic fibrosis. Respir Res 2015;16:103.
19. Prager B, Spampinato SF, Ransohoff RM. Sphingosine 1-phosphate signaling at the blood-brain barrier. Trends Mol Med 2015;21:354-63.
20. Baron JC. Perfusion thresholds in human cerebral ischemia: historical perspective and therapeutic implications. Cerebrovasc Dis 2001;11 Suppl 1:2-8.
21. Jefferson AL, Tate DF, Poppas A, et al. Lower cardiac output is associated with greater white matter hyperintensities in older adults with cardiovascular disease. J Am Geriatr Soc 2007;55:1044-8.
22. Hui S, Levy AS, Slack DL, et al. Sphingosine-1-Phosphate Signaling Regulates Myogenic Responsiveness in Human Resistance Arteries. PLoS One 2015;10:e0138142.
23. Balch WE, Roth DM, Hutt DM. Emergent properties of proteostasis in managing cystic fibrosis. Cold Spring Harb Perspect Biol 2011;3:a004499.
24. Roy R, Couriel JM. Secondary pulmonary hypertension. Paediatr Respir Rev 2006;7:36-44.

## Claims

1. A CFTR modulator for use in the prevention and/or treatment of a cerebrovascular condition wherein the CFTR modulator is at least one cyclopropane carboxamide derivative selected from the group consisting of lumacaftor, tezacaftor and pharmaceutically acceptable salts and solvates thereof.

2. The CFTR modulator for use of claim 1 wherein the CFTR modulator is lumacaftor or a pharmaceutically acceptable salt or solvate thereof.

3. The CFTR modulator for use of claim 1 or 2 wherein the cerebrovascular condition is reduced or impaired cerebral perfusion.

4. The CFTR modulator for use of claim 3 wherein the reduced cerebral perfusion is associated with a condition selected from the group consisting of heart disease, heart failure, subarachnoid haemorrhage, sudden sensoneurinal hearing loss, vascular dementia, arterial hypertension, ischemic stroke, hemorrhagic stroke, heart disease, diabetes and Alzheimer's disease.

## Patentansprüche

1. CFTR-Modulator zur Verwendung in der Verhinderung und/oder der Behandlung einer zerebrovaskulären Störung, wobei der CFTR-Modulator mindestens ein Cyclopropancarboxamid-Derivat ist, das aus der Gruppe, bestehend aus Lumacaftor, Tezacaftor und deren pharmazeutisch verträglichen Salzen und Solvaten, ausgewählt ist.

2. CFTR-Modulator zur Verwendung nach Anspruch 1, wobei der CFTR-Modulator Lumacaftor oder ein pharmazeutisch verträgliches Salz oder Solvat davon ist.

3. CFTR-Modulator zur Verwendung nach Anspruch 1 oder 2, wobei die zerebrovaskuläre Sörung eine verminderte oder beeinträchtigte zerebrale Perfusion ist.

4. CFTR-Modulator zur Verwendung nach Anspruch 3, wobei die verminderte zerebrale Perfusion mit einer Erkrankung im Zusammenhang steht, die aus der Gruppe, bestehend aus Herzkrankheit, Herzschwäche, Subarachnoidalblutung, plötzlichem Hörsturz, vaskulärer Demenz, arteriellem Bluthochdruck, Schlaganfall, ischämischem Schlaganfall, hämorrhagischem Schlaganfall, Herzkrankheit, Diabetes und Alzheimerscher Erkrankung, ausgewählt ist.

## Revendications

1. Modulateur CFTR utilisé dans la prévention et/ou le traitement d'une affection cérébrovasculaire, dans lequel le modulateur CFTR est au moins un dérivé cyclopropane carboxamide choisi dans le groupe constitué par lumacaftor, tezacaftor et leurs sels et solvates pharmaceutiquement acceptables.

2. Le modulateur CFTR pour l'utilisation selon la revendication 1, dans lequel le modulateur CFTR est lumacaftor ou un sel ou un solvant pharmaceutiquement acceptable de celui-ci.

3. Le modulateur CFTR utilisé selon la revendication 1 ou 2, dans lequel l'état cérébrovasculaire est une réduction ou une altération de la perfusion cérébrale.

4. Le modulateur CFTR utilisé selon la revendication 3, dans lequel la réduction de la perfusion cérébrale est associée à une affection choisie dans le groupe constitué par les maladies cardiaques, l'insuffisance cardiaque, l'hémorragie sous-arachnoïdienne, la perte soudaine d'audition neurosensorielle, la démence vasculaire, l'hypertension artérielle, l'accident vasculaire cérébral ischémique, l'accident vasculaire cérébral hémorragique, les maladies cardiaques, le diabète et la maladie d'Alzheimer.
